# EUROPEAN PATENT APPLICATION

(11) **EP 4 166 641 A1**
(43) Date of publication of application: **19.04.2023**
(21) Application number: 22157366.0
(22) Date of filing: 17.02.2022
(51) Int. Cl.: C12M 1/12

(54) **SYSTEMS AND METHODS FOR PROCESSING OLIVINE**

(30) Priority: 18.10.2021 US 202163256986 P; 19.11.2021 US 202163281575 P; 11.01.2022 US 202263298412 P
(71) Applicant: Project Vesta, PBC, San Francisco, CA 94114 (US)
(72) Inventor: ROMANIELLO, Stephen Justin, San Francisco, 94114 (US); LEY, Brian David, San Francisco, 94114 (US); ANDREWS, Margaret Grace, San Francisco, 94114 (US); WALWORTH, Nathan Gerard, San Francisco, 94114 (US); ISHOEY, Thomas, San Francisco, 94114 (US); GREEN, Tom Coxeter, San Francisco, 94114 (US); MONTSERRAT, Francesc, San Francisco, 94114 (US)
(74) Representative: Hamer, Christopher K.

(57) **Abstract**

The present disclosure provides a system for processing olivine. The system may comprise a reactor for accelerating olivine weathering. The reactor may comprise one or more chambers for housing one or more microbes, biological mediators, or enzymatic accelerants to facilitate the olivine weathering.

## Description

### CROSS REFERENCE

This application claims priority to U.S. Provisional Patent Application No. 63/256,986 filed on October 18, 2021, U.S. Provisional Patent Application No. 63/281,575 filed on November 19, 2021, and U.S. Provisional Patent Application No. 63/298,412 filed on January 11, 2022, each of which is incorporated herein by reference in its entirety for all purposes.

### BACKGROUND

Enhanced weathering or accelerated weathering may be used to remove atmospheric carbon dioxide by leveraging natural or artificially created minerals that can absorb and transform carbon dioxide into other substances through chemical reactions that may occur in the presence of water (e.g., in the form of rain, groundwater or seawater).

### SUMMARY

The present invention relates to a system for processing olivine. More specifically, the present invention relates to a system for processing olivine wherein the system comprises a reactor for accelerating olivine weathering, more particularly, the reactor may comprise one or more chambers for housing one or more microbes, biological mediators, or enzymatic accelerants to facilitate the olivine weathering. Enhanced weathering may be used in marine environments to increase ocean alkalinity and atmospheric CO2 uptake. In some cases, enhanced weather may involve adding processed minerals directly to the ocean or dumping the processed minerals on beaches where wave action can disperse the processed minerals into the target marine environment. Enhanced weathering may be used to reduce CO2 levels in the atmosphere while also counteracting ocean acidification.

Recognized herein are various limitations with current weathering methods, which may (i) involve long lead times and labor intensive procedures, (ii) impact ecological conditions (e.g., increasing water turbidity), and (iii) handling/processing of minerals that can pose additional ecological and health hazards via the possible ingestion, contact, or inhalation of fine grain of olivine mineral dust.

The present application relates generally to using olivine to achieve enhanced weathering and increase CO2 uptake on accelerated timescales. In one aspect, the present disclosure provides systems and methods for accelerating weathering. In another aspect, the present disclosure provides a bioreactor configured to accelerate dissolution of olivine for weathering applications. In a further aspect, the present disclosure provides systems and methods for enhancing material properties of olivine. For example, the present disclosure provides a way to optimize the surface area effect of fine olivine grains by utilizing a binding process to bind or encapsulate the olivine fines into pellets of a larger effective grain size which can be more safely handled and distributed in the environment. The ability to tailor the design of pellets composed of olivine mineral fines by using different binding agents or encapsulation processes provides the ability to tailor and control the porosity, permeability, and degradation characteristics of the olivine-containing pellets, controlling their dissolution behavior in the environment. The systems and methods disclosed herein may enable efficient processing and deployment of olivine for purposes of CO2 capture in marine environments.

In some embodiments, pelletized olivine aggregates formed from olivine fines may be prepared through a number of processes disclosed herein including [1] the use of a binding agent (such as but not limited to clay, polysaccharides, proteinaceous compounds, resin, plastic, glass, etc.) [2] via encapsulation of smaller olivine grains with said binding agent or other similar materials or [3] via the thermal fusion or physical compression of smaller olivine grains into larger aggregates.

In some embodiments, the pellets may be designed to either [4] remain substantially intact while maintaining improved surface area via internal porosity and permeability or [5] degrade in a controlled fashion to release fine olivine fines to the environment in a prolonged controlled fashion. In some cases, [6] the preparation of such pellets may allow olivine dissolution to occur substantially faster than would occur for equivalently sized solid pure olivine grains, thereby improving the process of carbon capture while minimizing the aesthetic, health, and ecological harm and challenges posed by working with and utilizing olivine mineral fines.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** schematically illustrates the long-term carbonate-silicate cycle, in accordance with some embodiments.
**FIG. 2** schematically illustrates the chemical processes by which olivine can be used to capture and sequester CO2, in accordance with some embodiments.
**FIG. 3** schematically illustrates the chemical reactions that enable CO2 capture and sequestration using olivine, in accordance with some embodiments.
**FIG. 4** schematically illustrates olivine and mixtures of sand and olivine, in accordance with some embodiments.
**FIG. 5** schematically illustrates one exemplary method for using olivine to remove atmospheric CO2 and increase ocean alkalinity, in accordance with some embodiments.
**FIG. 6** schematically illustrates the use of wave energy to cause sand grains and/or olivine grains to collide and break into smaller pieces, thereby facilitating the dissolution of olivine, in accordance with some embodiments.
**FIG. 7** schematically illustrates an example of how the oceans can be used for natural carbon storage, in accordance with some embodiments.
**FIG. 8** schematically illustrates an example of a bioreactor, in accordance with some embodiments.
**FIG. 9** schematically illustrates an example of a system for deploying olivine for coastal nourishment, in accordance with some embodiments.
**FIG. 10** schematically illustrates a computer system that is programmed or otherwise configured to implement methods provided herein.
**FIG. 11** schematically illustrates an exemplary reactor configured to intake seawater that may be mixed with olivine and microbial biomass to generate alkalinity.
**FIG. 12** schematically illustrates an exemplary protocol for measuring, recording, and verifying (MRV) carbon removal, in accordance with some embodiments.
**FIGs. 13 - 14** schematically illustrate a chemical reaction by which coastal carbon capture occurs when olivine is introduced to a target site, in accordance with some embodiments.
**FIGs. 15 - 20** schematically illustrate an environment in which olivine can be introduced for costal carbon capture, in accordance with some embodiments.
**FIGs. 21A** and **21B** schematically illustrates a plot of concentration of an alkaline material in a flux chamber as a function of time, in accordance with some embodiments.
**FIG. 22** schematically illustrates an overview of the porewater method, in accordance with some embodiments.
**FIG. 23** schematically illustrates a shrinking core model, in accordance with some embodiments.
**FIG. 24** schematically illustrates dissolution rates for olivine having various grain sizes, in accordance with some embodiments.
**FIG. 25** schematically illustrates an exemplary modeling approach for particle distribution, in accordance with some embodiments.
**FIG. 26** schematically illustrates dissolution rates for different mixtures of olivine having different mean grain sizes, in accordance with some embodiments.
**FIG. 27** schematically illustrates the impact of temperature and pH on olivine half-life, in accordance with some embodiments.
**FIG. 28** schematically illustrates examples of temporal factors and spatial factors that can vary for coastal ecosystems, in accordance with some embodiments.
**FIG. 29** schematically illustrates plots of the temporal heterogeneity of various characteristics for a target site, in accordance with some embodiments.
**FIGs. 30 - 32** schematically illustrate examples of factors to consider when calculating alkalinity fluxes, in accordance with some embodiments.
**FIG. 33** shows a summary of various approaches for measuring, recording, and verifying carbon capture and olivine dissolution at different scales of cost and complexity, in accordance with some embodiments.
**FIG. 34** schematically illustrates various reaction-transport modeling studies that may be used to simulate sediment porewater profiles, solid-phase chemistry, and benthic fluxes, in accordance with some embodiments.
**FIGs. 35 - 36** schematically illustrate various examples of sensors that can be used for measuring, recording, and verifying carbon capture and/or olivine dissolution, in accordance with some embodiments.
**FIGs. 37 - 38** schematically illustrate reactions that may occur when olivine sand is introduced to beaches, in accordance with some embodiments.
**FIG. 39** schematically illustrates an approach to calculating CO2 sequestration from alkalinity flux based on an expression of how water DIC storage changes as a function of increasing alkalinity, in accordance with some embodiments.
**FIG. 40** schematically illustrates a plot showing seawater age and the depth below sea water level of the Atlantic Ocean as a function of latitude (degrees North), in accordance with some embodiments.
**FIG. 41** schematically illustrates an exemplary coastal enhanced weathering (CEW) life cycle analysis, in accordance with some embodiments.
**FIG. 42** schematically illustrates an example of a carbon payback period that can be realized using the methods and systems disclosed herein, in accordance with some embodiments.
**FIG. 43** schematically illustrates various plots showing exemplary olivine dissolution kinetics for olivine particles having different grain sizes, in accordance with some embodiments.
**FIG. 44** schematically illustrates the effects of secondary carbonate precipitation on CO2 capture efficiency, in accordance with some embodiments.
**FIG. 45** schematically illustrates the effects of secondary clay formation on CO2 capture efficiency, in accordance with some embodiments.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The term "real time" or "real-time," as used interchangeably herein, generally refers to an event (e.g., an operation, a process, a method, a technique, a computation, a calculation, an analysis, a visualization, an optimization, etc.) that is performed using recently obtained (e.g., collected or received) data. In some cases, a real time event may be performed almost immediately or within a short enough time span, such as within at least 0.0001 millisecond (ms), 0.0005 ms, 0.001 ms, 0.005 ms, 0.01 ms, 0.05 ms, 0.1 ms, 0.5 ms, 1 ms, 5 ms, 0.01 seconds, 0.05 seconds, 0.1 seconds, 0.5 seconds, 1 second, or more. In some cases, a real time event may be performed almost immediately or within a short enough time span, such as within at most 1 second, 0.5 seconds, 0.1 seconds, 0.05 seconds, 0.01 seconds, 5 ms, 1 ms, 0.5 ms, 0.1 ms, 0.05 ms, 0.01 ms, 0.005 ms, 0.001 ms, 0.0005 ms, 0.0001 ms, or less.

### Overview

To avoid the worst effects of climate change, we must rapidly remove billions of tons of CO2 from the atmosphere. There are currently no proven methods for doing so. There is an urgent need to identify carbon removal methods that are permanent, scalable, and economical.

The earth's natural long-term carbonate-silicate cycle is how our planet has naturally captured CO2 over thousands of years. Over millennia, rain falling on exposed volcanic rock causes it to weather, which removes CO2 from the atmosphere as bicarbonate dissolved in water. This water flows to the oceans, where marine calcifying organisms incorporate the CO2 into their shells and skeletons. When they die, they form ocean sediment, ultimately becoming limestone.

The systems and methods of the present disclosure enable the acceleration of Earth's natural carbon removal method of rock weathering, through the extraction, processing (e.g., crushing and/or grinding), and transportation of volcanic mineral olivine to coastlines for dispersal or distribution. In some embodiments, wave energy may cause the olivine particles to collide, grinding each other up and dramatically accelerating the weathering process. In other embodiments, a bioreactor may be used to enhance and facilitate olivine dissolution. In either case, carbon dioxide may be removed from the atmosphere as the olivine breaks down.

Coastal Carbon Capture, also known as Coastal Enhanced Weathering, can be categorized as a negative emission technology (NET) that removes and stores CO2 on long timescales (tens to hundreds of thousands of years). The process aims to accelerate the natural chemical weathering of the mineral olivine by spreading large amounts of ground olivine-containing rock onto coastlines or bioreactors positioned on or near such coastlines, where it can dissolve in seawater, thereby increasing the rate of CO2 absorption by the ocean.

The present disclosure provides systems and methods for accelerating the Earth's natural carbon removal method of rock weathering, by extracting the volcanic mineral olivine, grinding it into sand, and deploying it on coastlines or in bioreactors located on said coastlines. There, wave energy may cause the olivine particles to collide, grinding each other up and dramatically accelerating the weathering process. Carbon dioxide is removed from the atmosphere as the olivine breaks down.

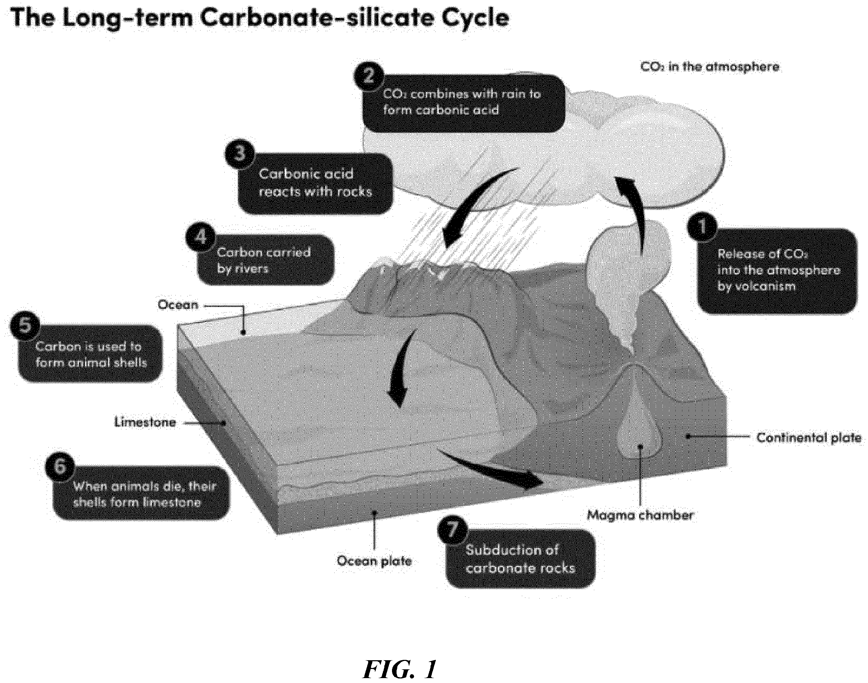

**FIG. 1** illustrates the long-term carbonate-silicate cycle. The illustrated reaction is the foundation of the Long-Term Inorganic Carbon Cycle, which has been occurring for billions of years, stabilizing Earth's atmospheric CO2 concentrations, and in turn enabling life to thrive. In fact, carbon dioxide removal (CDR) through natural rock weathering consumes ~1 gigaton of CO2 every year. Natural CO2 removal through rock weathering may be achieved through the following steps:
1. Rain falls on volcanic rocks, slowly dissolving them.
2. Carbonic acid dissolved in the rainwater reacts with silicate from the rocks, shifting the equilibrium from carbonic acid to bicarbonate.
3. This bicarbonate flows to the ocean.
4. Marine organisms use this as calcium carbonate in their shells and skeletons; when they die this becomes limestone.
5. Limestone formed at the ocean floor eventually subducts, locking up the carbon for millions of years.

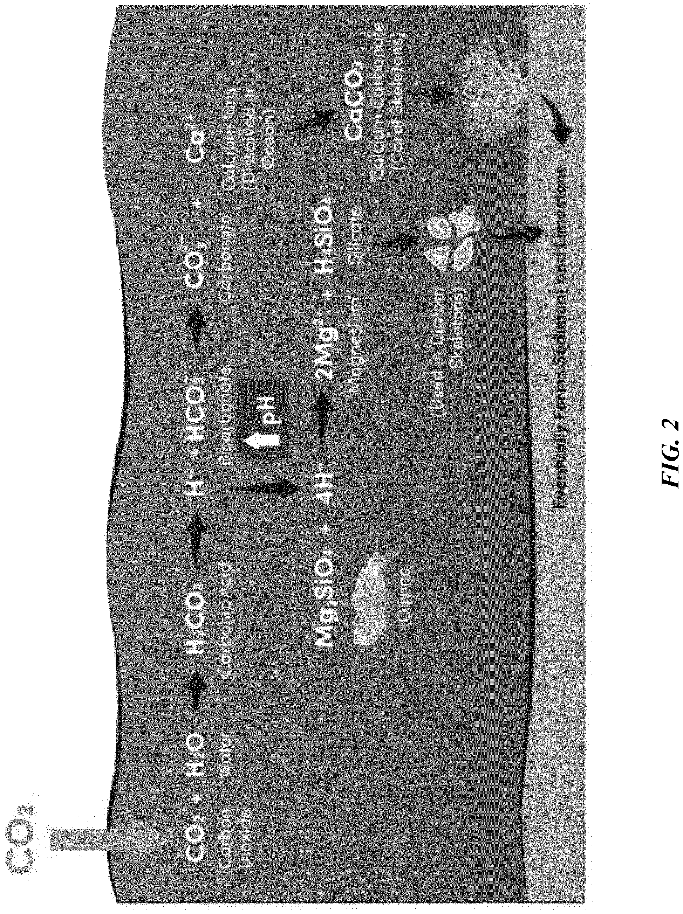

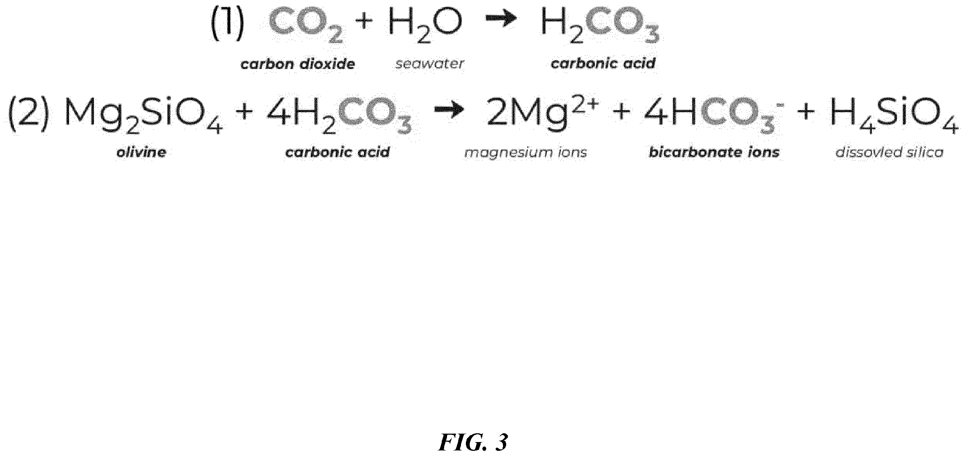

**FIG. 2** illustrates the chemical processes that are enabled by olivine to perform CO2 capture and sequestration. **FIG. 3** illustrates the chemical reactions that enable CO2 capture and sequestration using olivine. As the olivine dissolves, its products are magnesium ions (the second most abundant ion in the ocean behind sodium), silicate (used by diatoms to build their skeletons) and dissolved carbon. Referring to **FIG. 2** and **FIG. 3**, when olivine dissolves in water, it drives the illustrated reaction, thus increasing CO2 uptake, raising pH, and generating alkalinity. As a result, this process has the potential co-benefit of counteracting ocean acidification. Ocean acidification is the process by which increasing atmospheric CO2 dissolves in seawater, which reduces pH (increasing acidity) (upper reaction in diagram below). This reduces the ability of calcifying organisms like corals to grow and produce exoskeletons, or shells. As shown in **FIG. 2** and **FIG. 3**, dissolving olivine in water sequesters hydrogen ions into dissolved silicate (H4SiO4), a molecule that can be used by diatoms, an important photosynthesizing algae that fixes carbon dioxide and forms the base of food web.

Unfortunately, natural chemical weathering happens too slowly to balance human CO2 emissions and is already accounted for in Earth's present-day carbon budget. The systems and methods disclosed herein can be deployed and implemented to accelerate this natural process to remove at least a gigaton of atmospheric CO2 per year on a global scale.

### Accelerating a Natural Process

Coastal Carbon Capture can be used to accelerate Earth's natural CO2 removal process. At current rates, the natural process of rock weathering through rainfall needs to be sped up by at least 100 times to absorb the CO2 emitted by human activity. Coastal Carbon Capture (CCC) can accelerate the process by using wave energy and/or bioreactors to break down olivine. The rock containing the olivine may be placed in high-energy coastal environments, where wave energy mechanically activates the rock, causing collisions which remove the silica coating and efficiently grind the olivine down. The olivine sand then dissolves in the water at a rate orders of magnitude faster than in the geological process.

Coastal Carbon Capture may comprise the following steps:
1. Extract olivine and efficiently grind it. Olivine is a green volcanic silicate mineral. It is highly abundant and found near the surface all over the world. Olivine rocks can be efficiently crushed to sand and gravel grain sizes.
2. Transport olivine to a nearby coastal area. The olivine sand may be transported (e.g., by rail, ship, or truck) to a nearby coastal shelf sea. There, it may be spread in a thin layer.
3. Wave energy may accelerate CO2 removal. Wave energy and currents may further grind down the olivine. Grain-on-grain collisions can cause fine fraction particles (< 10 microns) to form, which then weather very rapidly. The olivine may consequently dissolve in the water rapidly, accelerating the reaction which removes CO2 from the ocean/atmosphere.

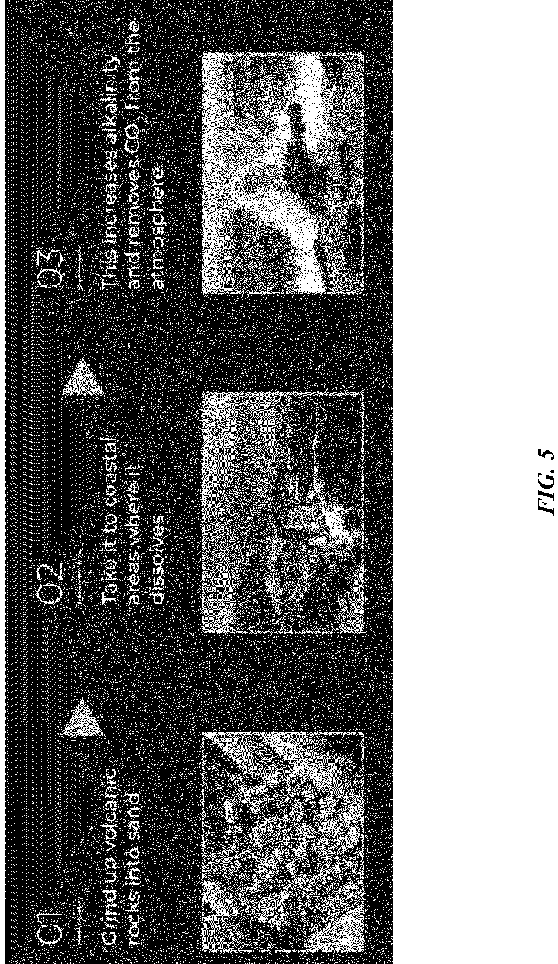

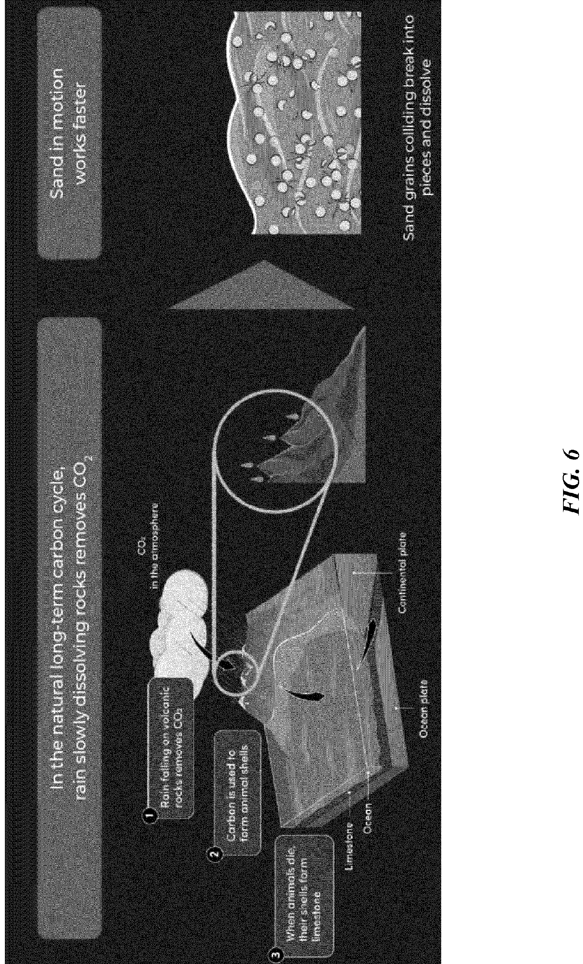

**FIG. 5** illustrates one exemplary method for using olivine to remove atmospheric CO2 and increase ocean alkalinity. As shown in **FIG. 6**, wave energy may cause sand grains and/or olivine grains to collide and break into smaller pieces, thereby facilitating the dissolution of olivine.

### Olivine

Olivine is a common, naturally occurring volcanic mineral, with physical properties similar to quartz. Olivine may be 23% denser than quartz sand (1.43 tons/cy). Olivine sand is a clean upland sand that is 25% denser than silicate sand, and that can be artificially milled to match native grain size. Darker and lighter olivine sources allow for color matching to the native sand color.

Olivine can be processed to yield fine olivine grains with increased surface area exposure, which can speed up dissolution and the CO2 capture process. The olivine dissolution and CO2 capture process can also be accelerated by (i) using reactive and soluble minerals, (ii) increasing an amount of surface area of the olivine grains that is exposed to a solvent (e.g., water or seawater), (iii) enhancing fluid-mineral interactions, and/or (iv) reducing or eliminating secondary mineral coatings or the formation of secondary weathering products (e.g., precipitation of secondary clays and carbonate phases).

Once deployed on a beach, olivine can capture 20 times more carbon than is emitted in the mining, milling, shipping, and beach nourishment operation. 1 ton of olivine sand can capture up to 1.25 tons of carbon in ideal situations. Since it takes olivine several decades to weather, it will provide effective coastal protection during long-term beach nourishment cycles.

One type of olivine may be white olivine, or Forsterite (Mg2SiO4). This is a magnesium rich form of olivine, known as Forsterite (Mg2SiO4). Weathering 1 ton of olivine can remove up to 1.25 tons of CO2 from the atmosphere.

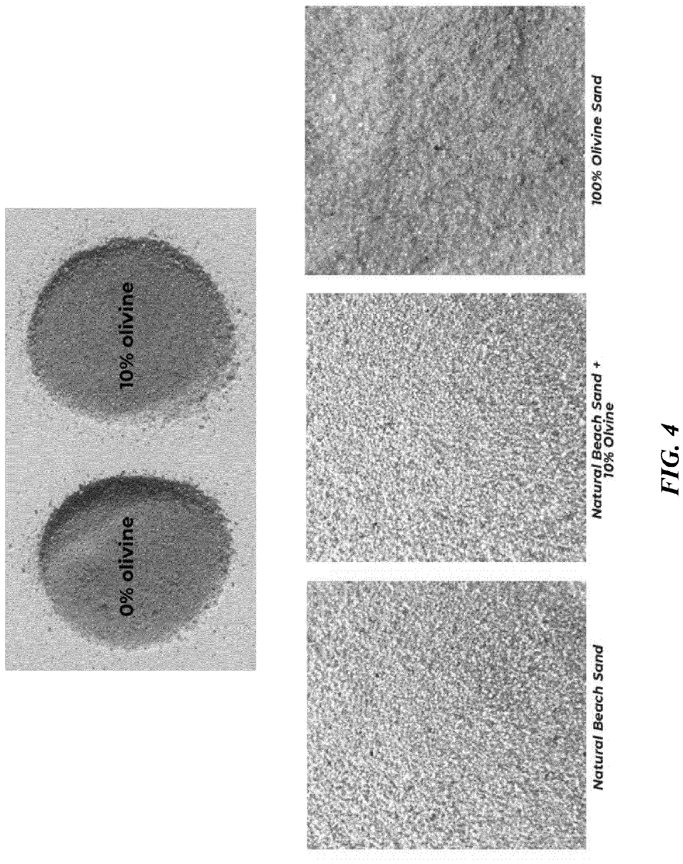

**FIG.** 4 illustrates olivine and mixtures of sand and olivine. In some cases, the olivine may be mixed with natural beach sand before the olivine is deployed at a target site. The mixture comprising the olivine may comprise at least about 5%, 10%, 15%, 20%, 25%, or 30% olivine by weight or volume. In some cases, the mixture may comprise more than 30% olivine by weight or volume. In some cases, the mixture may comprise less than 5% olivine by weight or volume. In some cases, the mixture may comprise between about 5% and about 30% olivine by weight or volume.

Beneficial applications of olivine include, for example, Marsh Restoration, Coastal Engineering/Hazard Mitigation, and CO2 Capture. As described above, olivine sand can react with water and CO2. CO2 may dissolve in water to form carbonic acid. Olivine dissolution may consume the carbonic acid to yield soluble bicarbonate ion (baking soda). This can: Lower atmospheric CO2, Neutralize ocean acidification, and Support the growth of calcifying organisms (Corals, etc).

In some cases, olivine sand may be provided to municipalities to augment the sediment used in beach nourishment projects. As the sand weathers, it captures carbon. The sand and its placement are paid for by carbon credits. Therefore, the greater the percentage of olivine sand mixed into the native sand, the cheaper the beach nourishment project. A 10% mix would mean a -10% decrease in sand cost. Through the use of olivine sand, beach nourishment can play a critical role in reversing climate change and ocean acidification if the right mix of native and olivine sand is used. At scale, over a billion tons of CO2 could be removed from the atmosphere every year.

Using wave action to break down the olivine is critical to the life cycle. Past analyses have shown that grinding the olivine down to <100µm size is highly energy-intensive. However, grinding the olivine down to >300µm gravel requires far less energy. Using the natural, free energy of water movement to break the olivine down further reduces the milling and grinding energy to a small proportion of the total.

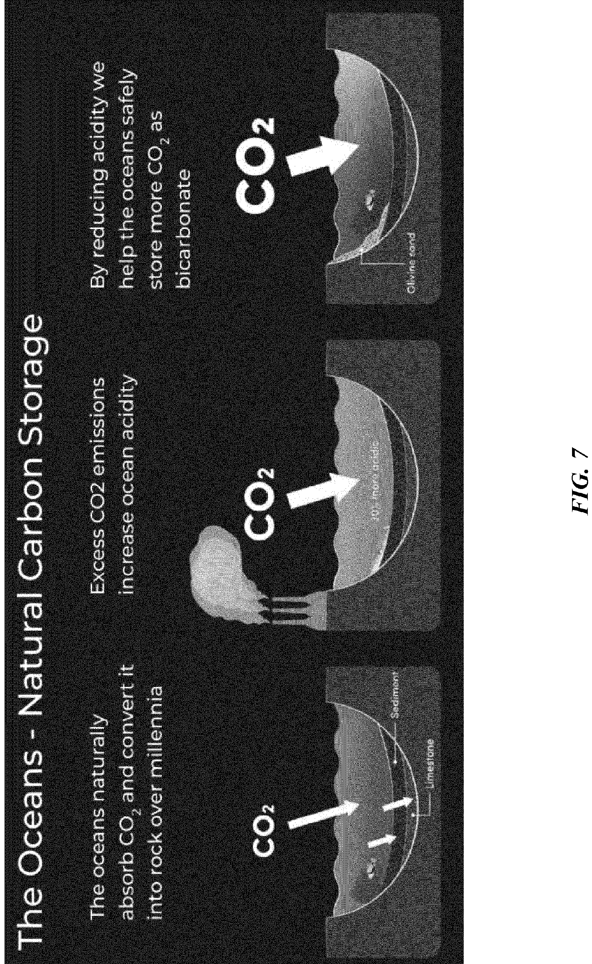

Co-Benefits of the methods described herein include, for example: reduction in Ocean Acidification (OA). Ocean acidification has been shown to damage marine ecosystems. Olivine increases local pH levels, potentially mitigating the adverse effects of OA on marine ecosystems. This could potentially improve aquaculture and fishery yields, bolstering local economies at deployment sites as well as reversing damage to marine ecosystems. FIG. 7 illustrates an example of how the oceans can be used for natural carbon storage. Excess CO2 emissions may increase ocean acidity. The systems and methods of the present disclosure may be implemented to reduce acidity and help the oceans safely store more CO2 as bicarbonate.

Co-Benefits of the methods described herein may also include, for example: support for diatom growth. Diatoms, marine photosynthetic microalgae, are a cornerstone of marine food webs and capture CO2 through photosynthesis, creating 40% of oxygen on Earth. Diatom abundance has decreased as CO2 has risen, partly due to decreasing concentrations of silicate in the ocean due to damming of rivers limiting sediment flow. Since olivine is primarily magnesium silicate, the increase in silicate concentrations from olivine dissolution may lead to diatom growth. Increases in diatoms would increase the health of marine ecosystems and support photosynthetic carbon capture. Other co-benefits can include, for example, supporting coastal communities facing enhanced coastal erosion and sea level rise by supplying a low-cost source of sediment (which may be paid for using carbon credits).

### Mechanics of Weathering

In some cases, grain-on-grain collisions may rapidly break down the olivine. Olivine grains may be reduced in size due to mechanical activation. In some cases, the motion of the water in which the olivine is provided may cause surface abrasions. In some cases, the surface of the olivine grains may be mechanically activated to enable and/or enhance CO2 uptake and de-acidification.

### Sensors

In some cases, one or more sensors may be used to track, monitor, and quantify olivine dissolution, CO2 uptake, and ocean de-acidification. The measurements obtained using the sensors may be used to adjust the preparation or processing of the olivine, the optimal properties of the olivine, the location in which the olivine is introduced, the rate at which the olivine is introduced, a method by which the olivine is distributed, or a configuration/form in which the olivine is distributed.

In some cases, the one or more sensors may be used to monitor and track: Olivine Dissolution Rates (Porewater Geochemistry, Benthic Flux Measurements), Impacts on Water Chemistry, Ecological Response, Fate, Transport and Bioaccumulation of Cr and Ni, and/or Formation of Secondary Weathering Products (Chrysotile, Carbonates, Clays). In some cases, an isotopic tracer along with [Si] and [ALK] measurements may be used to track olivine dissolution and simultaneous precipitation of secondary clays and carbonate phases. The sensors may also be used to track olivine grains over time [ie physical tracking of olivine using dyes / tracers, etc.].

### Reaction-Transport Modeling

In some cases, simulation models may be used to optimize the preparation or processing of the olivine, the properties of the olivine, the location in which the olivine is introduced, the rate at which the olivine is introduced, a method by which the olivine is distributed, or a configuration/form in which the olivine is distributed. The models may be configured to simulate sediment porewater profiles, Water column and Porewater Chemistry (e.g., pCO2, DIC, pH, DO, TA, Nutrients, Metals, DOC), solid-phase chemistry and benthic fluxes, Secondary Clay Mineralogy, and Cr and Ni speciation and cycling. In some cases, the models may be used for simulation of the effects of spreading layers of olivine sand of varying thicknesses on a target beach sediment with one or more known biological, chemical, or ecological properties or characteristics.

In some cases, the models may be used simulate or predict Olivine Dissolution Rates, study the impacts of Temperature of Olivine Dissolution & Respiration, track impacts on seawater pH, track Air-Sea Water Gas CO2 Fluxes, monitor and evaluate fate and transport of Cr and Ni, assess the impacts on benthic invertebrates/meiofauna, track sediment characteristics (e.g., changes in mineralogy, formation of secondary carbonate/clays), monitor the impacts of Meteorology and Hydrology (e.g., temperature, wind speed/direction, precipitation, salinity, turbidity, current), or simulate Physical Sediment Transport and/or Olivine Redistribution.

### Method of Removing Atmospheric Carbon

[1] In one aspect, the present disclosure provides a method for removing atmospheric carbon using olivine dissolution technologies. The method may be implemented using natural wave energy (i.e., in situ coastal enhanced weathering (CEW)) and/or applied energy (i.e., closed-system artificial weathering of olivine in a man-made reactor or a sand pit resulting from coastal nourishment projects or dredging).

### Bioreactor

In another aspect, the present disclosure provides systems and methods to accelerate olivine dissolution reactions in a bioreactor. The bioreactor may comprise one or more internal chambers for housing or storing olivine for any amount of time. The bioreactor may comprise one or more openings or inlets for receiving olivine. The olivine may comprise pure olivine or a mixture of olivine and sand or other elements or materials. The olivine may be unprocessed. Alternatively, the olivine may be processed or pre-processed (e.g., by grinding).

In some cases, the bioreactor may comprise one or more mechanical components for mechanical weathering of the olivine. The mechanical weather may cause grain-on-grain collisions that may rapidly break down the olivine. Olivine grains may be reduced in size due to mechanical activation. In some cases, the bioreactor may be in fluid communication with water. In some cases, the bioreactor may comprise an internal chamber for housing the water and/or the olivine. The motion of the water in which the olivine is introduced may cause surface abrasions. In some cases, the surface of the olivine grains may be mechanically activated to enable and/or enhance CO2 uptake and ocean de-acidification.

In some cases, the mechanical components may comprise one or more gears or actuators for inducing a motion that cause mechanical weathering of the olivine. The motion may be a linear motion, or a rotational motion, or any combination thereof.

In some cases, the bioreactor may comprise a chamber for housing one or more microbial products or other biological mediators and/or enzymatic accelerants that can help to further break down the olivine. The chamber housing the microbial products may be in fluid communication with (i) the chamber housing the olivine, (ii) the chamber that is in fluid communication with the ocean water, or both (i) and (ii).

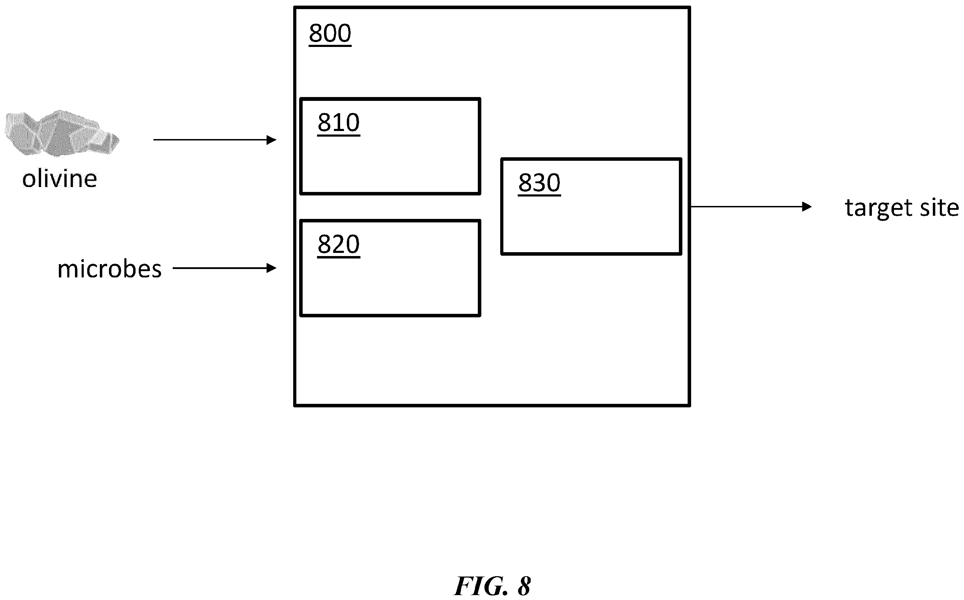

**FIG. 8** schematically illustrates an example of a bioreactor 800. The bioreactor 800 may be configured to receive olivine, which olivine may or may not be pre-processed (e.g., by grinding or crushing). The olivine may be stored within a first chamber 810 of the bioreactor 800 for further processing. In some cases, the bioreactor 800 may comprise a second chamber 820 configured to house one or more microbial products or other biological mediators and/or enzymatic accelerants that can help to further break down the olivine. In some cases, the bioreactor 800 may comprise a third chamber 830. The third chamber 830 may comprise a reaction chamber in fluid communication with the first chamber 810 and the second chamber 820. The third chamber 830 may be used to break down the olivine using the microbial products or other biological mediators and/or enzymatic accelerants. Once broken down, the processed olivine may be provided to a target region external to the bioreactor 800 to facilitate CO2 capture and sequestration.

### Microbes

[2] In another aspect, the present disclosure provides a method for enhanced dissolution of olivine through (1) microbial products or other biological mediators and (2) enzymatic accelerants. The method may comprise enhancing dissolution of olivine in an olivine reactor or sand pit resulting from coastal nourishment projects or dredging through: microbial products or other biological mediators; and enzymatic accelerants.

In another aspect, the present disclosure provides systems and methods for olivine dissolution by microorganisms, biological organisms, and/or biological mechanisms. The dissolution of olivine in water may consume protons and increase alkalinity, thereby removing dissolved aqueous CO₂. This aqueous CO₂ removal may induce additional CO₂ to be taken up from the atmosphere and subsequently converted to bicarbonate, carbonate, and/or solid-phase carbonate minerals, thereby sequestering carbon for long time periods [1]. Scaling and accelerating this process has the potential to mitigate climate change and ocean acidification.

In some cases, the reactors disclosed herein may be designed to grow particular microbial strains in an aqueous medium in order to induce metabolic states that can accelerate olivine dissolution. In circum-neutral pH naturally oxygenated waters, ferric iron (Fe²⁺) contained within olivine may be rapidly oxidized to ferrous iron (Fe³⁺) by oxygen even in the absence of microbial activity [2]. This oxidized iron can form a surface layer of Fe-oxyhydroxides on the mineral surface of olivine which can shield portions of its total surface area from contact with solution [3]. These Fe-oxyhydroxide surface coatings can, in some cases, impede olivine dissolution [4].

For the vast majority of organisms, Fe is a required nutrient and a key component of many cellular proteins. However, most Fe in naturally oxygenated waters at circum-neutral pH is insoluble and typically complexed to particles. Certain microorganisms can produce organic compounds known as siderophores to mobilize and acquire insoluble iron on mineral surfaces [2], which can concurrently increase olivine mineral dissolution rates [3]. However, the production of siderophores may be regulated by Fe availability to the cells. As more Fe is acquired by the cells (i.e. the less iron-limited they are), siderophore production may decrease, which may slow dissolution rates. Hence, in order to sustain and scale siderophore production over extended time periods in large reactors, numerous interacting factors may be optimized:
1. The most optimized biological strains (these may be identified through a series of experiments, and in some cases, custom strains may be generated either through directed evolution or genetic editing).
2. The optimal ratios of microbial to siderophore concentrations given certain growth conditions.
3. Olivine dissolution optimization as a function of particle size/surface area and flow rate from biomass generation.
4. Optimized long-term growth conditions for sustained siderophore production.
5. Optimized reactor design series including appropriate materials to avoid silicate and trace metal contamination.
6. Development of mathematical model to optimize siderophore production yield, biomass generation, and olivine dissolution.
7. Development of appropriate parameters to track to feed into the model.
8. Development of any treatment of seawater effluent prior to returning to the ocean.
9. Development of a model that calculates CO₂ sequestration from the addition of alkalinized seawater to the local ocean region.

### System Design

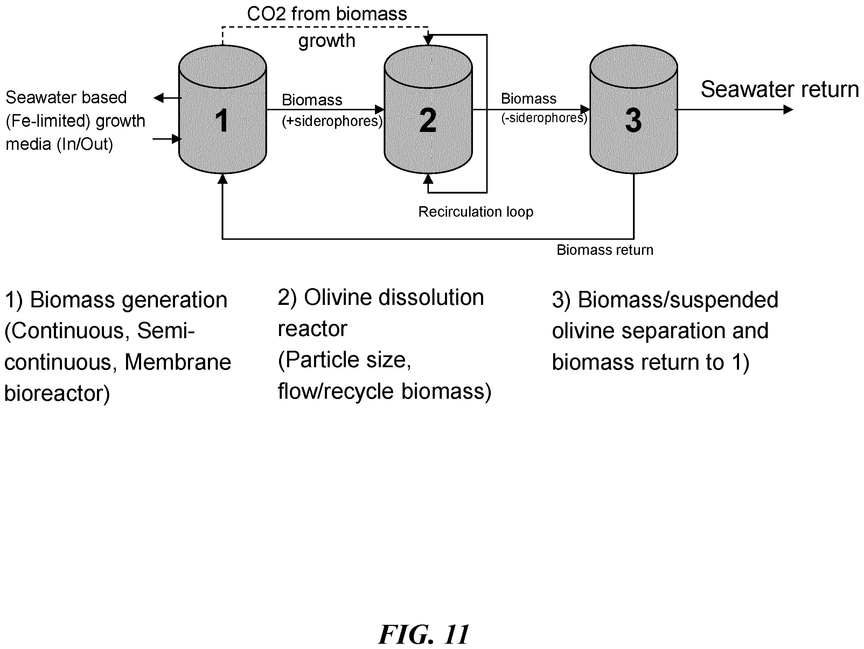

Referring to **FIG. 11**, the illustrated reactor may be configured to intake seawater that may be mixed with olivine and microbial biomass to generate alkalinity. Depending on the source, seawater may have to be pretreated prior to reacting with olivine. This reaction may also result in trace metals, microbial biomass, and other reaction products mixing in with the seawater medium. In order to sequester carbon at scale, this alkaline seawater may be returned to the ocean to induce atmospheric CO₂ uptake. Importantly, the return of this seawater containing the certain constituents must meet local regulations. Another microbially-mediated treatment process that returns effluent to the environment may involve anaerobic digestion. This may be a good reference to understand regulations for effluent from microbial processes.

In some cases, the reactor may comprise a first chamber for biomass generation (e.g., continuous, semi-continuous, membrane bioreactor), a second chamber for olivine dissolution (e.g., to optimize particle size and flow/recycle biomass), and a third chamber for biomass/suspended olivine separation (and for biomass return to the first chamber). In some cases, sea water based growth media may flow into and/or out of the first chamber. In some cases, CO2 from biomass growth may be provided to the second chamber. The second chamber may also receive biomass with siderophores from the first chamber. The second chamber may provide, in some cases, a recirculation loop for the biomass. The second chamber may be configured to provide the biomass without the siderophores to the third chamber. In some cases, the third chamber may be configured to return biomass to the first chamber. In some cases, the third chamber may be configured to provide the olivine and/or the biomass to seawater.

The present disclosure effectively provides methods and designs for sustained microbial growth and siderophore production for olivine dissolution over long time periods. Current methods are unable to accelerate olivine dissolution with microbes for reaction volumes exceeding a few hundred milliliters, unlike the systems and methods disclosed herein. The presently disclosed systems and methods have been developed with the specific engineering and regulatory requirements needed to build large-scale, flow-through reactors with seawater that will then be returned to the local environment. The present disclosure effectively provides systems and methods for optimizing (1) strain selection for olivine dissolution, (2) grain size, (3) culturing conditions with natural seawater, and (4) the materials for the growth chamber.

### Optimizing Olivine

[3] In another aspect, the present disclosure provides systems and methods for optimizing olivine for use in the field. Such optimization may comprise, for example, determining the optimal grain size distribution of olivine sand for use in carbon capture (covers combinations of grain size, color, blends, includes modeling grain size for specific sites); and physically and/or chemically processing the olivine (e.g., using machinery) to prepare one or more batches of carbon negative sand suitable for beach nourishment.

### Preparing Olivine

[4] In another aspect, the present disclosure provides systems and methods for preparing olivine grains for optimal dissolution. The olivine grains may be part of a source material comprising olivine sand or a mixture comprising olivine sand and local sand.

In some cases, the method may comprise crushing raw olivine to yield an optimal grain size and aggregating raw or crushed olivine into pellets. Such processing of the olivine may enhance dissolution of olivine, thereby accelerating the carbon capture process. In some cases, the method may further comprise selecting, milling, and/or matching olivine sand to native sediment for the purpose of coastal protection, beach nourishment and carbon capture.

In another aspect, the present disclosure provides carbon-negative sand and processes for producing the same. The carbon-negative sand may comprise specially selected, prepared, and blended mineral particles which [1] are suitable to mitigate coastal erosion along coasts [2] react with carbon dioxide (CO₂) and/or dissolved carbonic acid [H₂CO_{3]} to produce bicarbonate [HCO₃⁻], [CO₃²⁻] ions, and/or solid-phase carbonate minerals [Ca, Mg]CO₃, thus [3] simultaneously mitigating the impacts of climate change, ocean acidification, and coastal erosion.

In order to be suitable for use in coastal erosion, beach nourishment, and marsh restoration projects, [4] manufactured carbon-negative sand and blends thereof should closely match the texture, grain size distribution, and color of native sediment. Thus, the processes for preparing carbon negative sand may be tailored such that the carbon-negative sand and blends thereof produce and achieve these specific pre-determined properties.

In order to mitigate the impacts of carbon dioxide (ie. be "carbon negative"), [5] carbon negative sand may be manufactured from a mafic or ultramafic inorganic mineral or rock (typically naturally occurring olivine, basalt, serpentinite, brucite, wollastonite, or industrial-produced mineralequivalents such as slag). These minerals [6] may react with water and carbon dioxide and/or carbonic acid to produce bicarbonate ion as a reaction product, thereby decreasing the acidity of the surrounding fluid and converting the harmful carbon dioxide or carbonic acid into environmentally beneficial bicarbonate or carbonate ion. An example describing the reaction of forsterite olivine (Mg2SiO4) is provided below, although other minerals and rocks described in this disclosure result in equivalent reactions converting carbonic acid to bicarbonate ion:

CO₂ + H₂O → H₂CO₃

Mg₂SiO₄ + 4 H₂CO₃ → 2 Mg²⁺ + 4 HCO₃⁻ + H₄SiO₄

In order to simultaneously achieve the engineering requirements of sand and sediment for coastal engineering, while preserving the chemical reactivity with carbon dioxide described above, [7] it may be necessary to prepare specially prepare the reactive mineral components and then blend these reactive components in specifically determined ratios with inert native or allochthonous sand and sediment to achieve the desired chemical, engineering, and aesthetic properties. This process is described in greater detail below:

The process for preparing the CO₂ - reactive component of carbon negative sand begins with a feedstock of mafic or ultramafic rocks, minerals, or their industrially produced equivalents. A list of potential feedstocks is provided in Table 1.

**Table 1**. Potential feedstock materials for the production of the CO₂ - reactive component of carbon negative sand.

| |
|---|
| **Naturally Occurring Rocks:** Peridotite, Dunite, Harzburgite, Lherzolite, Basalt, Gabbro, Komatiite, Serpentine |
| **Naturally Occurring Minerals:** Olivine, Forsterite, Fayalite, Enstatite, Pyroxene, Serpentine, Brucite |
| **Industrial Byproducts:** Blast-furnace Slag, Steel-furnace Slag, Basic-oxygen-furnace Slag, Electric-arc-furnace Slag, Ladle Slag. |

The preparation of the CO₂ - reactive components of carbon negative sand may proceed via two critical steps:
(1) The feedstock may be crushed and/or milled to increase the reactive surface area of the feedstock and provide fresh reactive surfaces for the reaction with carbon dioxide. Because the reaction occurs only on the surfaces of the grains and within microcracks created by the crushing/milling process, it is essential to increase the available reaction surface area to the maximum extent possible in order to maximize the material reactivity.
(2) The crushed and milled material may then be size sorted using sieves, gravity separation, air classifiers, to provide a final product with a mean grain size and overall grain size distribution which meets the engineering specifications of the coastal engineering project. In some cases, this process may be designed to exclude excessive fine-grained material and/or coarser particles, to produce an intermediate grain-size material which closely matches the size distribution of the native sediment in the geographic region receiving the carbon negative sand. As an alternative to preparing batches of CO₂ - reactive components individually, it is also possible to presort the material into various predetermined size ranges and then select and/or blend from these preclassified separates to achieve an overall blend suitable for use in a specific project.
   In most cases or all cases, the CO₂ - reactive component may be further modified via mixing non-reactive sediment in order to achieve a combination of texture, color, and density, and engineering properties suitable for use in coastal engineering products. To achieve this, a third step may be required:
(3) The CO2-reactive mineral component may be blended with one or more chemicallyinert sand and sediment materials to achieve a predetermined ratio providing the specified properties. A list of common non-reactive feedstocks is provided in **Table 2**. Blends consisting of 1-50% reactive component may be most common, but other variations of this invention, higher or lower fractions of the reactive component may be used.

**Table 2. Potential feedstock materials for the non-reactive component of carbon-negative sand.**

| |
|---|
| **Naturally Occurring Native Sediment at the Project Site:** May include native sand, silt, clay, organic matter and gravel |
| **Naturally Occurring Rocks/Sediment:** Silicate Beach Sand, Carbonate Beach Sand, Dredged Sediment, Crushed Sandstone, Crushed Limestone, Carbonate Ooids |
| **Naturally Occurring Minerals:** Quartz, Feldspar, Aragonite, Calcite, Garnet, Magnetite, Hematite, Limonite, Ilmenite, Smectite, Chlorite, Biotite, Muscovite, Rutile, Zircon and Monazite |

The goal of blending the reactive- and non-reactive sediment components is to achieve a final bulk product meeting various regulatory, aesthetic and engineering requires including color, texture, grain size, density, cohesion, hardness, etc. To realize a blended mixture between the reactive and non-reactive components of the carbon-negative sand mixture, the individual components may either be brought together in advance, or in alternate embodiments, separately conveyed or placed onto the project site in such a manner to realize an appropriate mixture over the duration of the project (ie. mixing between components occurs via the physical motion of sediment driven by conveyors, heavy equipment, and/or waves and tides).

Although enhanced silicate weathering may be implemented in some instances, none of the prior work has [8] recognized and provided methods for the manufacture of engineered carbon-negative materials with specific suitable colors, grainsize, density, and reactivity required to meet specific regulatory, aesthetic, and engineering requirement for coastal engineering. Moreover, [9] prior work has completely neglected the engineering of these specific attributes in order to create safe and successful materials for coastal engineering applications. [10] No existing material used in present coastal engineering applications provides additional public and ecological benefits due to CO₂ capture, alkalinity generation, and the mitigation of ocean acidification. Likewise, [11] no prior approaches have realized the simultaneous benefits derived from manufacturing carbon-negative materials which are suitable for coastal engineering, thus providing a dual benefit of mitigating coastal erosion and sea level rise.

### Carbon Credits

[5] In another aspect, the present disclosure provides systems and methods for measuring, recording and verifying (MRV) carbon removal as well as ecological effects of olivine sand in carbon removal over large coastal areas. Such measurements, recording, and verification of carbon removal may be performed using one or more remote sensors, protocols for the use of such sensors (benthic flux chambers), and algorithms to interpret the data obtained and derived from the sensor readings. The presently disclosed systems may be used to prove the dissolution of olivine and the subsequent capture of CO2 based on the dissolution of the olivine. In some cases, the systems and methods disclosed herein may be implemented to predict dissolution (e.g., by utilizing one or more models for simulating or predicting olivine dissolution at a target site).

The present disclosure also provides software configured to calculate CO2 consumption and to measure/record/verify carbon removal. The software may be configured to determine CO2 consumption and/or carbon removal based on one or more sensor readings and/or one or more input parameters. The one or more input parameters may relate to, for example, the physical or chemical properties of the olivine used, the ratio of olivine to sand, the location in which the olivine is distributed, an amount of olivine distributed to a target site, or the manner/configuration in which the olivine is distributed (e.g., method of distribution, form of distribution, or spatial characteristics of the distribution). In some cases, the sensor readings may comprise measurements of alkalinity, pCO2, pH, and/or water temperature.

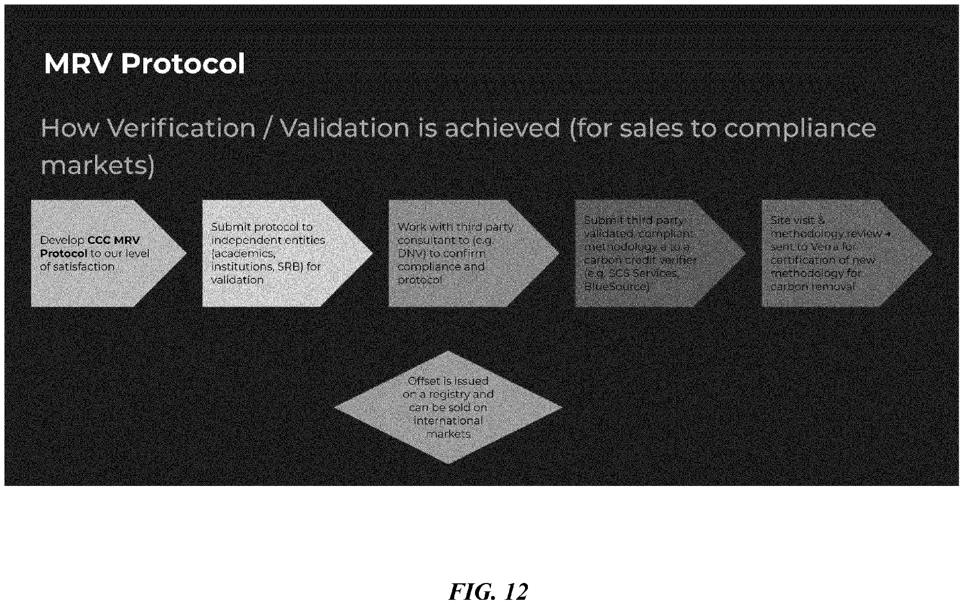

In one aspect, the present disclosure provides methods and protocols for measuring, recording, and verifying (MRV) carbon removal (e.g., by way of coastal carbon capture using olivine). Referring to **FIG. 12**, the protocols may be submitted to independent, third-party entities (e.g., academics, institutions, etc.) for validation. Once validated, the protocols may be implemented by an individual or an entity. Additional third-party entities may ensure and confirm compliance. The individuals or entities implementing the MRV protocols may then submit the third-party verified, compliant methodologies to a carbon credit verifier, and an offset or a credit may be issued on a registry for sale in domestic and/or international markets.

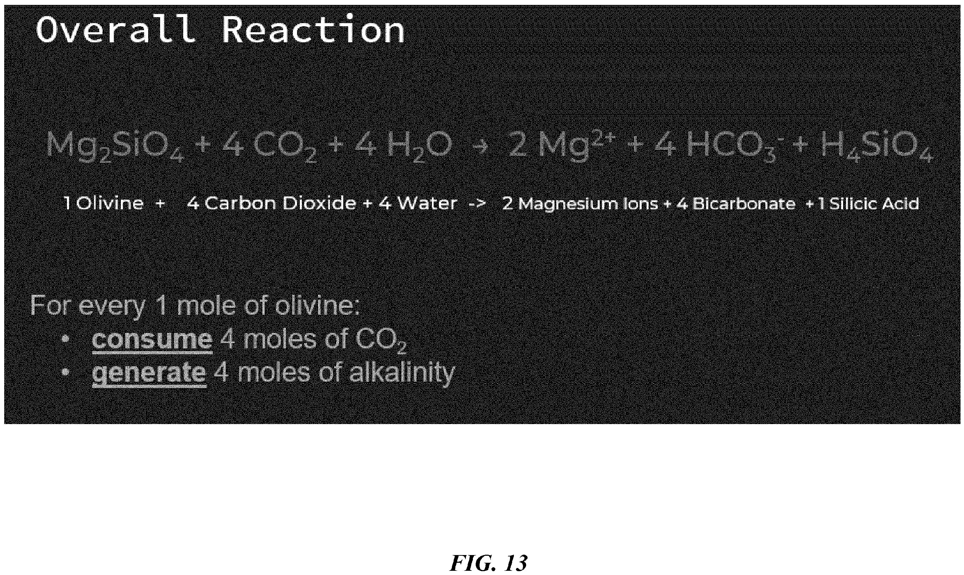

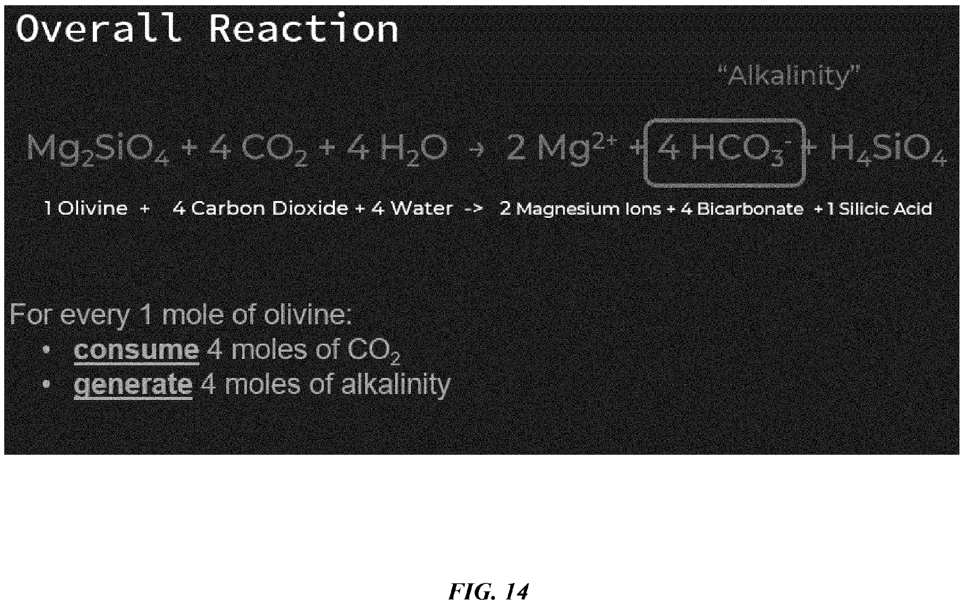

**FIGs. 13 - 14** and **FIG. 37** show a reaction representing the chemical mechanism by which coastal carbon capture can occur when olivine is introduced to a target site (e.g., by adding olivine sand to beaches and mixing the olivine sand with the native beach sand). For every 1 mol of olivine (which olivine may comprise or exhibit any of the physical or chemical properties or compositions described herein), 4 mols of carbon dioxide may be consumed or captured, and 4 mols of an alkaline material (bicarbonate or HCO₃⁻) may be generated.

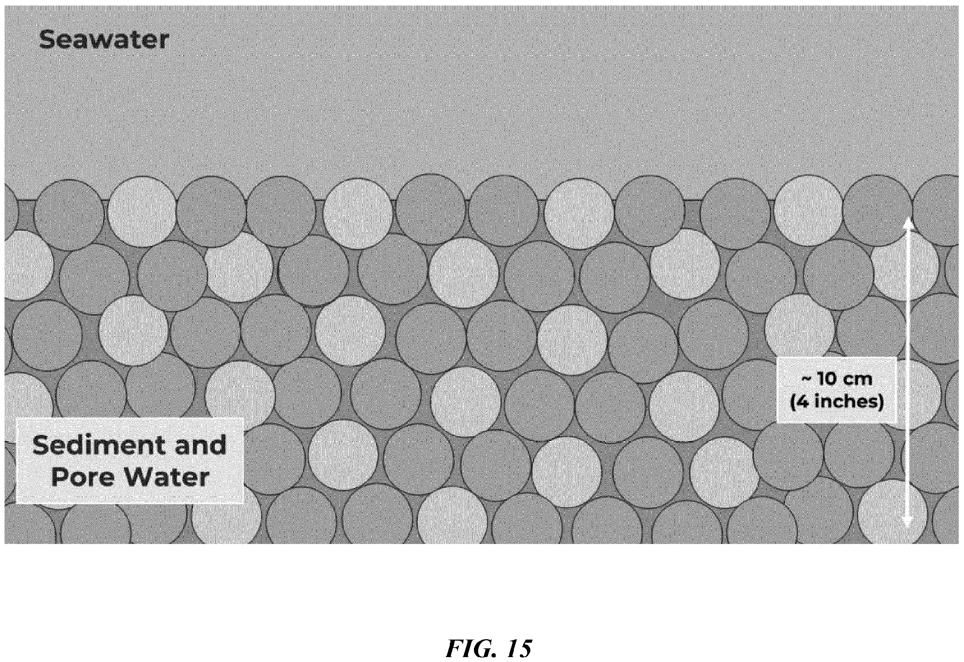

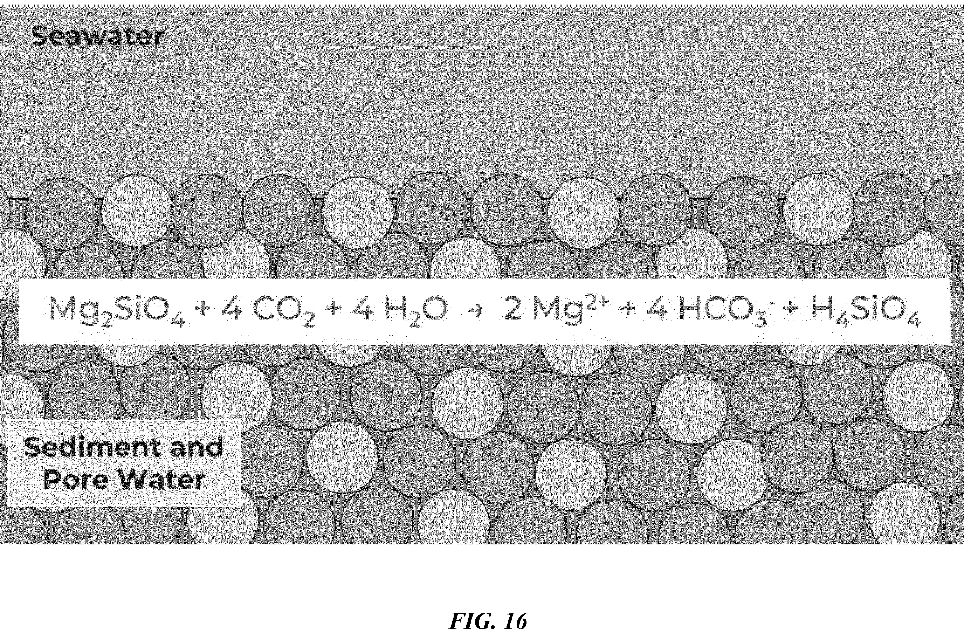

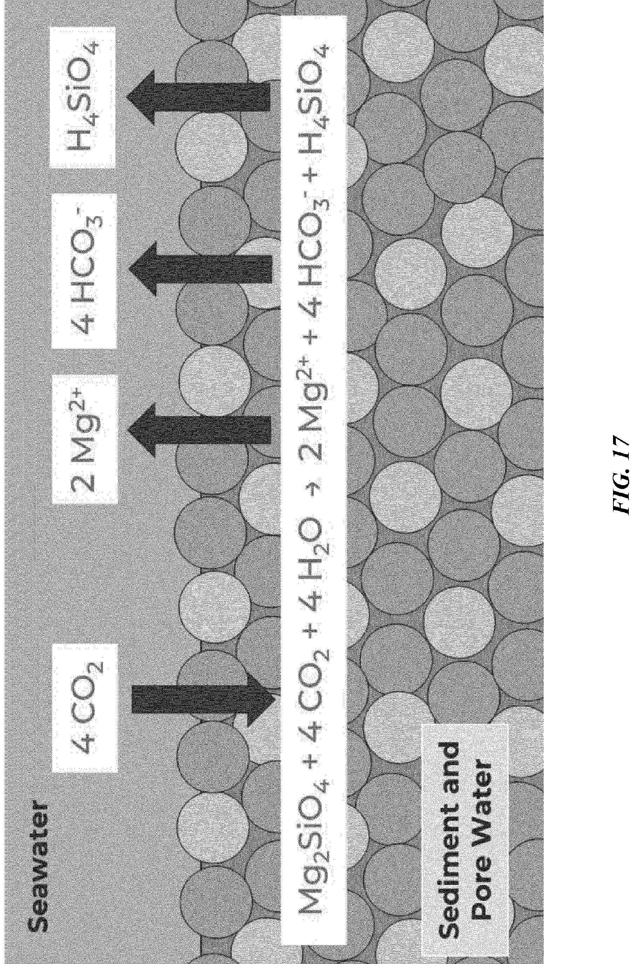

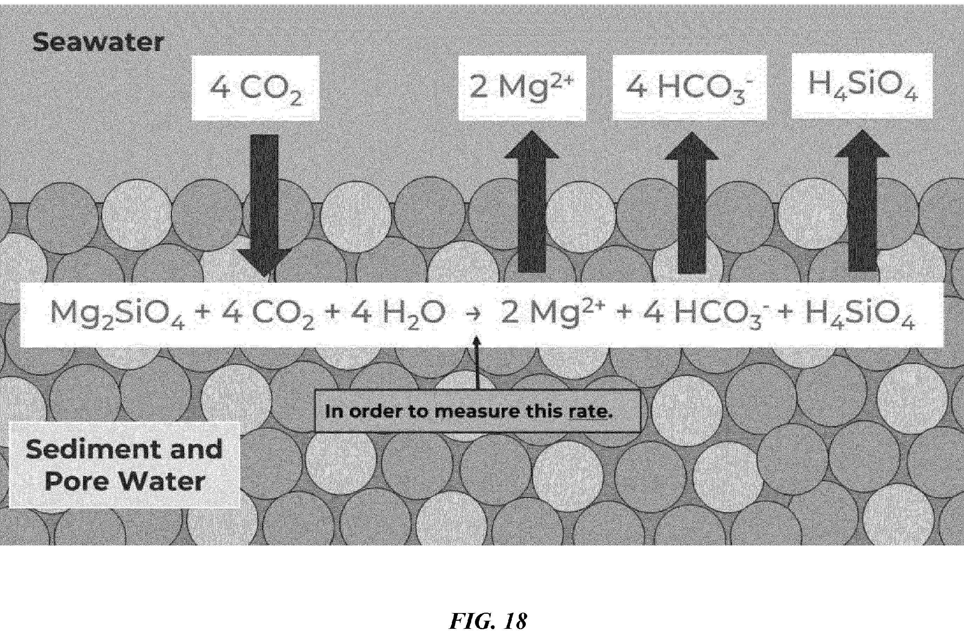

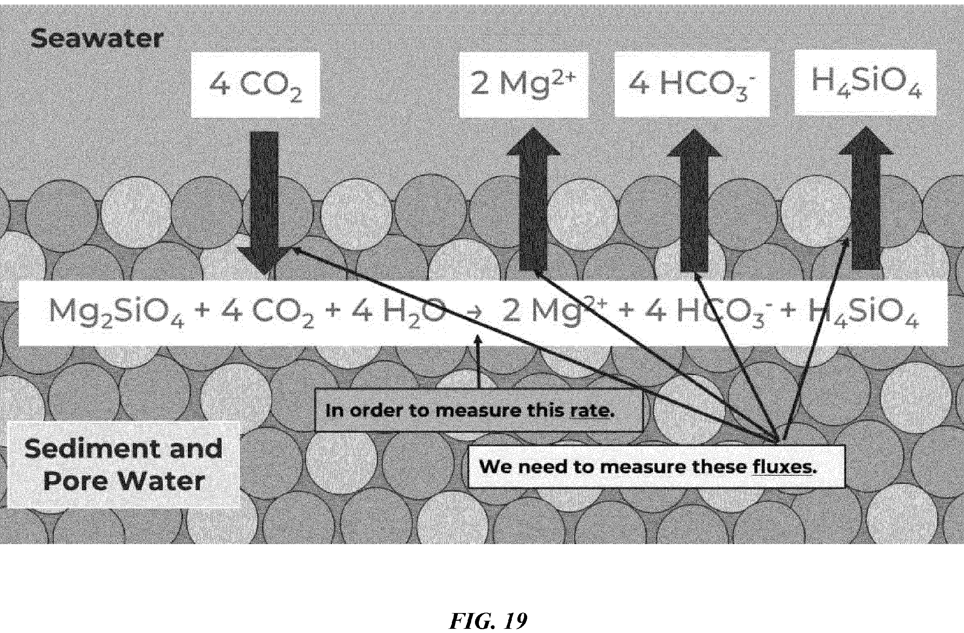

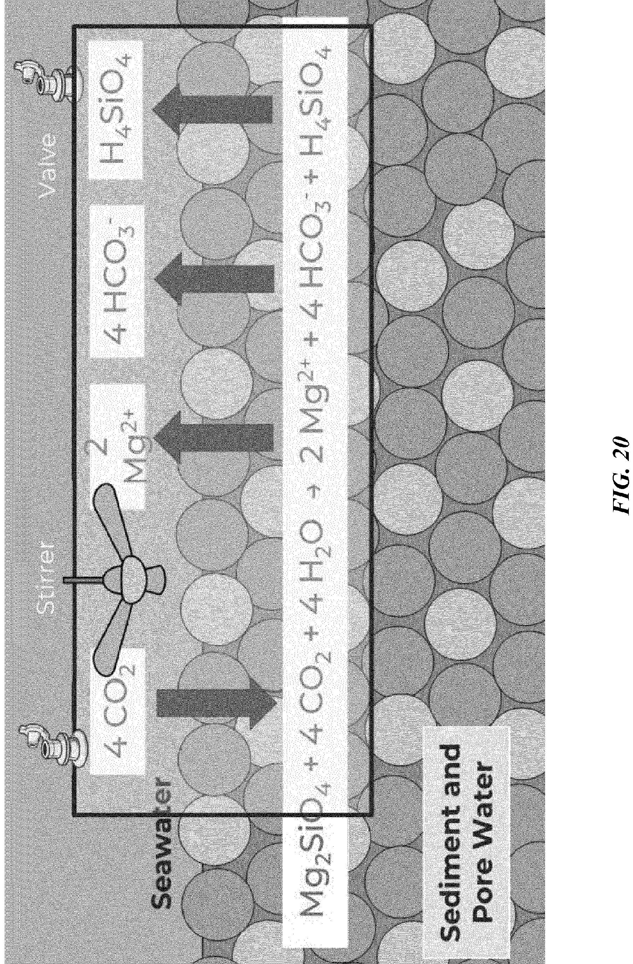

**FIGs. 15 - 20** show an environment comprising a first layer comprising seawater and a second layer comprising sediment and pore water. The sediment and pore water may span a dimension of about 10 centimeters (cm) or about 4 inches (in). The reactions shown in and described in relation to **FIGs. 13 - 14** and **FIG. 37** may drive the capture of carbon dioxide (e.g., CO2 in the atmosphere or in the seawater) and the release of magnesium, bicarbonate, and silicic acid. The rate of the overall reaction may be determined based on the fluxes associated with CO2 capture and the subsequent release of magnesium, bicarbonate, and silicic acid.

In some non-limiting embodiments, one or more stirrers may be used to facilitate the carbon capture process (e.g., by physically mixing the seawater containing olivine sediments with the medium comprising the carbon dioxide). The medium may comprise a liquid medium (e.g., seawater) or a gaseous medium (e.g., air comprising atmospheric carbon dioxide). In some non-limiting embodiments, one or more valves may be used to control and regulate an outflow of any products of the reaction (e.g., the magnesium, bicarbonate, and/or silicic acid).

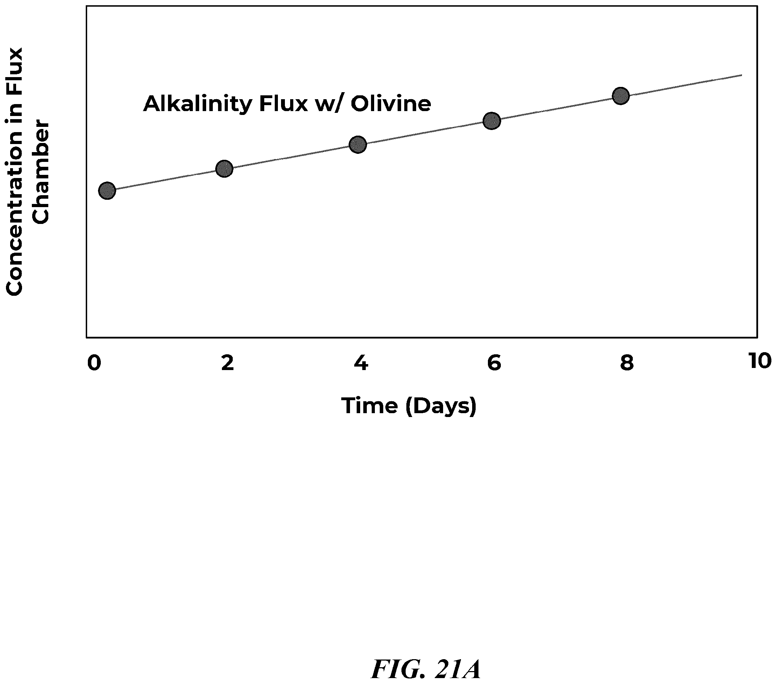

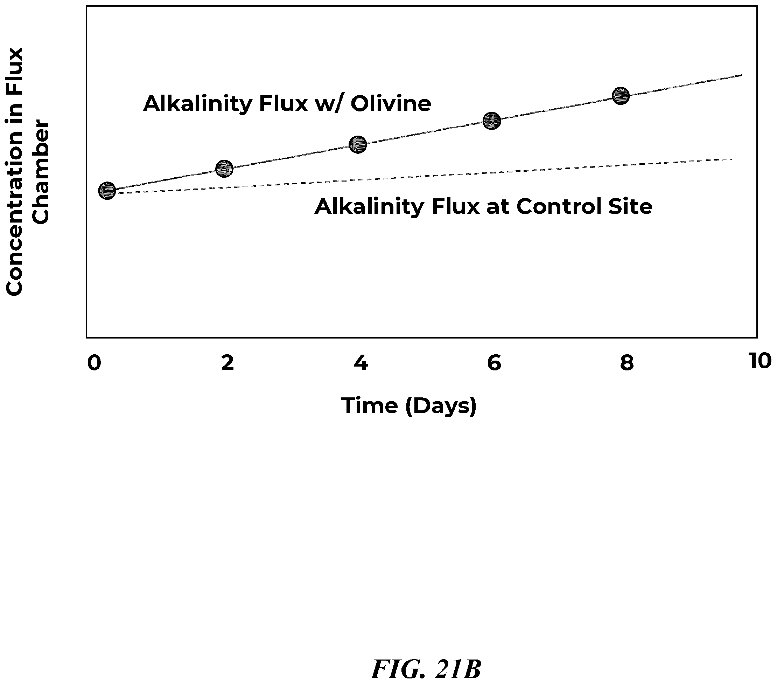

**FIG. 21A** illustrates a plot of a change in concentration of an alkaline material in a flux chamber as a function of time when the olivine-based carbon capture reactions of the present disclosure are used. The alkalinity of the medium in which the alkaline material is present may gradually increase over time. In some cases, the alkalinity may increase by a factor of 1.1 to 10 or more over a period of 1 to 10 days. As shown in **FIG. 21B**, the alkalinity flux with olivine increases more rapidly over time than the alkalinity flux at a control site in which olivine is not introduced. The alkalinity flux may correspond to the slope of the lines shown in these plots, and may have units of change in concentration of the alkaline material per unit time.

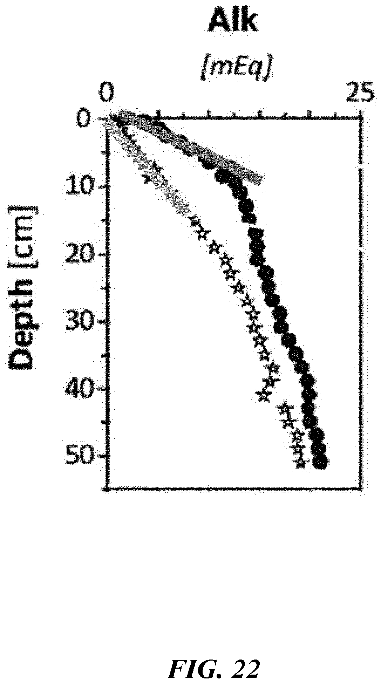

**FIG. 22** shows an overview of the porewater method for determining flux of an alkaline material that is produced. The porewater method may be based on Fick's first law of diffusion. The flux of the alkaline material may be determined based on a diffusion coefficient (representing area per unit time) and a dC/dZ value (representing a change in concentration or amount of substance per unit volume, as a function of position or dimensional length). With olivine present in porewater, a larger flux may be observed (steeper slope and bigger flux), whereas when olivine is not present, a smaller flux is observed (shallower slope and smaller flux).

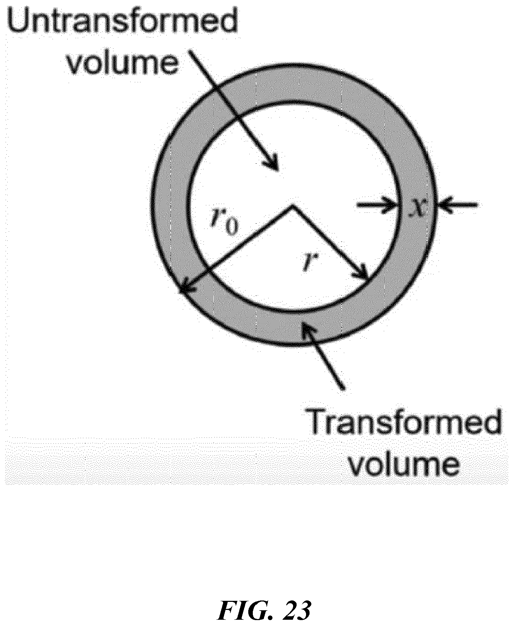

**FIG. 23** shows a shrinking core model comprising a transformed volume and an untransformed volume of an exemplary olivine particle. The shrinking core model may be represented by X = 1 - [1 - R/(ρ^{∗}d))^{∗}t]^3, where X is the fraction of olivine reacted, R is a function of pH and temperature, p is molar density, d is starting diameter, and t represents time. One assumption for this model is that the particles are a perfect sphere.

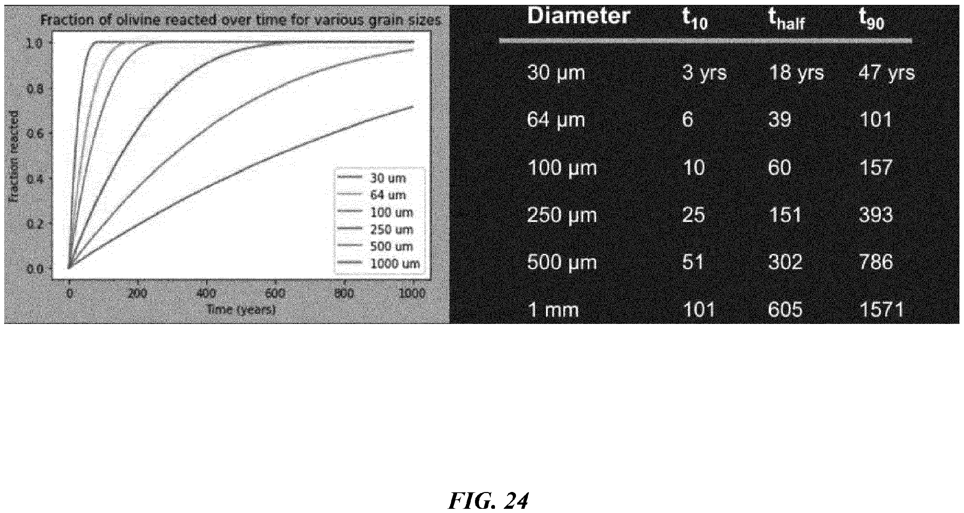

**FIG. 24** shows dissolution rates for olivine having various grain sizes. The fraction of olivine reacted over time may increase more rapidly for olivine particles having smaller grain sizes. For example, for olivine with a 30 micrometer grain size, 10% of the olivine may react within 3 years, 50% of the olivine may react within 18 years, and 90% of the olivine may react within 47 years. In contrast, for olivine with a 64 micrometer grain size, 10% of the olivine may react within 6 years, 50% of the olivine may react within 39 years, and 90% of the olivine may react within 101 years. The rates shown generally correspond to static dissolution rates based on inorganic chemical processes, and may not include acceleration of weathering due to wave energy and biotic processes. The systems and methods of the present disclosure may be implemented to optimize dissolution rates by (1) inducing or imparting wave energy with a specific characteristic (e.g., periodicity, intensity, motion path, etc.) and/or (2) modifying the biotic processes used to enhance olivine dissolution.

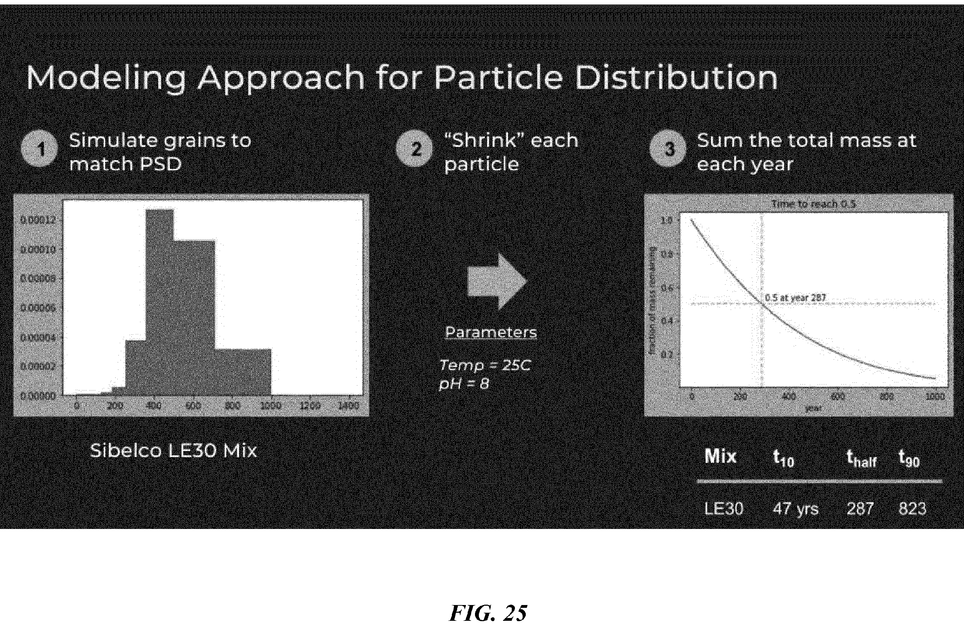

**FIG. 25** shows an exemplary modeling approach for particle distribution. In one step, the modeling approach may comprise simulating grains to match a desired particle size distribution. The modeling approach may further comprise shrinking each particle over a time period (e.g., to simulate dissolution or erosion of one or more portions or layers of each particle), and thereafter summing the total mass of the particles and/or the total amount dissolved or eroded at various time points in order to determine the fraction of mass remaining as a function of time.

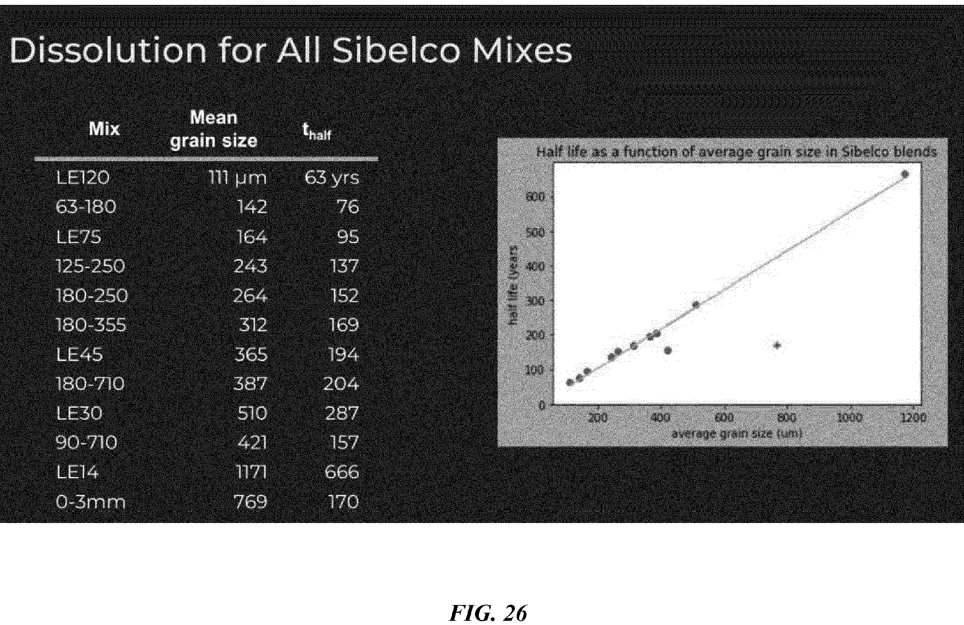

**FIG. 26** shows dissolution rates for different mixtures of olivine having different mean grain sizes. In general, olivine mixtures with smaller mean grain sizes may have a shorter half-life (i.e., a shorter time period for half of the olivine mixture to dissolve under a particular set of conditions).

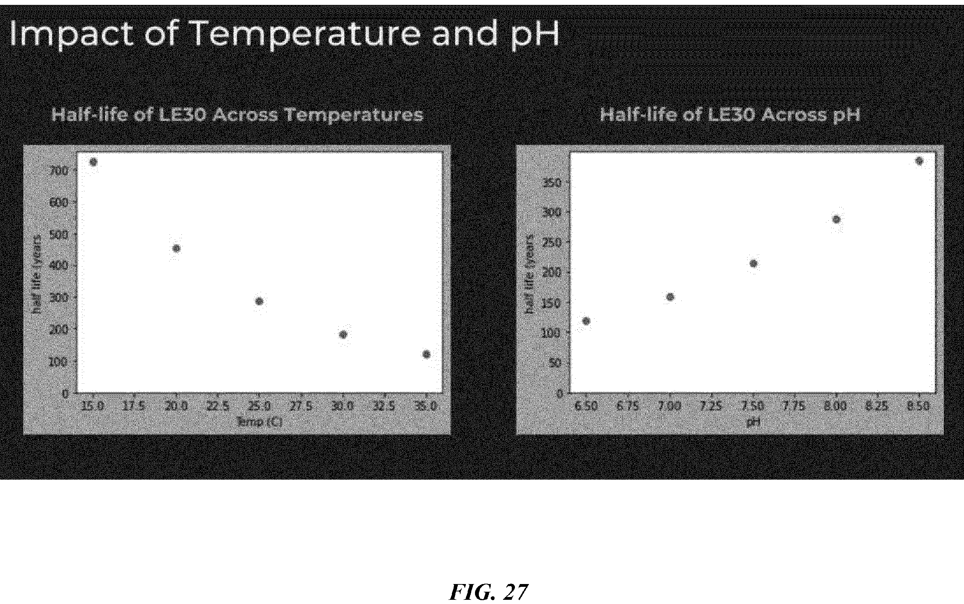

**FIG. 27** shows the impact of temperature and pH on half-life. In some cases, the half-life for olivine dissolution may decrease as temperature increases. In some cases, the half-life for olivine dissolution may increase as pH increases. In some embodiments, the systems and methods disclosed herein may be implemented to optimize temperatures and pHs for dissolution environments to shorten the half-life for olivine dissolution. In other embodiments, the systems and methods disclosed herein may be implemented to identify candidate locations with optimal temperatures and pHs for olivine dissolution environments to shorten the half-life for olivine dissolution.

The systems and methods disclosed herein may be configured or implemented to overcome challenges relating to spatial and temporal heterogeneity, low signal/noise ratios due to slow dissolution of olivine, and variation in secondary mineral product generation. The systems and methods disclosed herein may also help to overcome verification challenges such as, for example, objective criteria for acceptance and verification of carbon credit sales, and how to influence such criteria to adapt them to coastal weathering processes.

The present disclosure provides methods for performing MRV (i.e., the measurement, recording, and verification) of carbon capture and/or olivine dissolution. In some cases, the methods may comprise establishing a treatment site and a reference or baseline site. The methods may further comprise measuring alkalinity fluxes and/or other parameters at a network of discrete locations. In some cases, this may be repeated over time as olivine dissolves over a series of time points. The methods may further comprise interpolating in time and space to determine an overall dissolution rate across an area or a volume of the site. In some cases, the overall dissolution rate across the site may be determined in part using a numerical reaction transport model that provides a fit to the measured data.

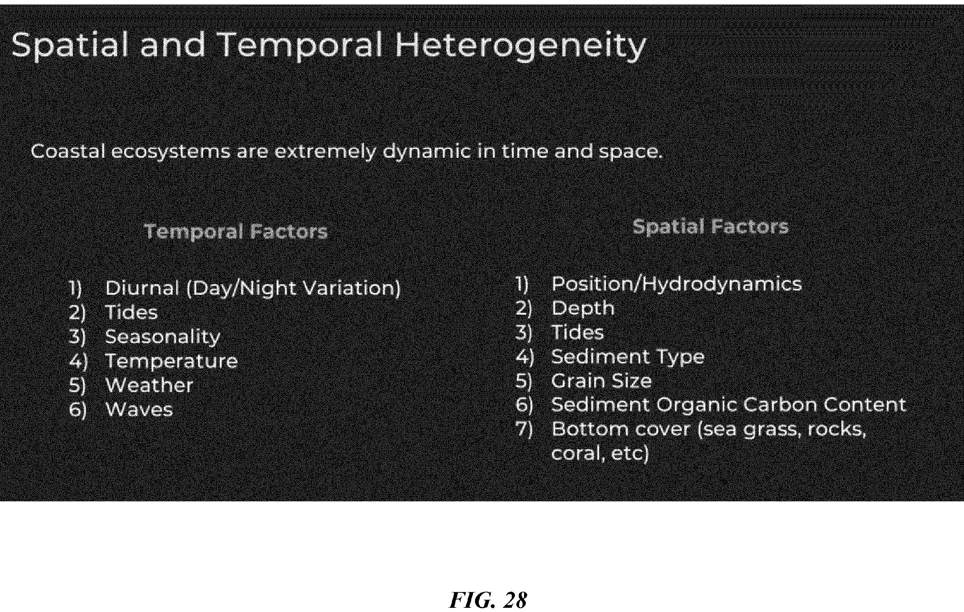

**FIG. 28** shows examples of temporal factors and spatial factors that can vary for coastal ecosystems, many of which are extremely dynamic. Temporal factors may include, for example, diurnal factors (i.e., day/night variations), tides, seasonality, temperature, weather, and/or waves. Spatial factors may include, for example, position and hydrodynamics, depth, tides, sediment type, grain size, sediment organic carbon content, and/or bottom cover (e.g., sea grass, rocks, coral, etc.).

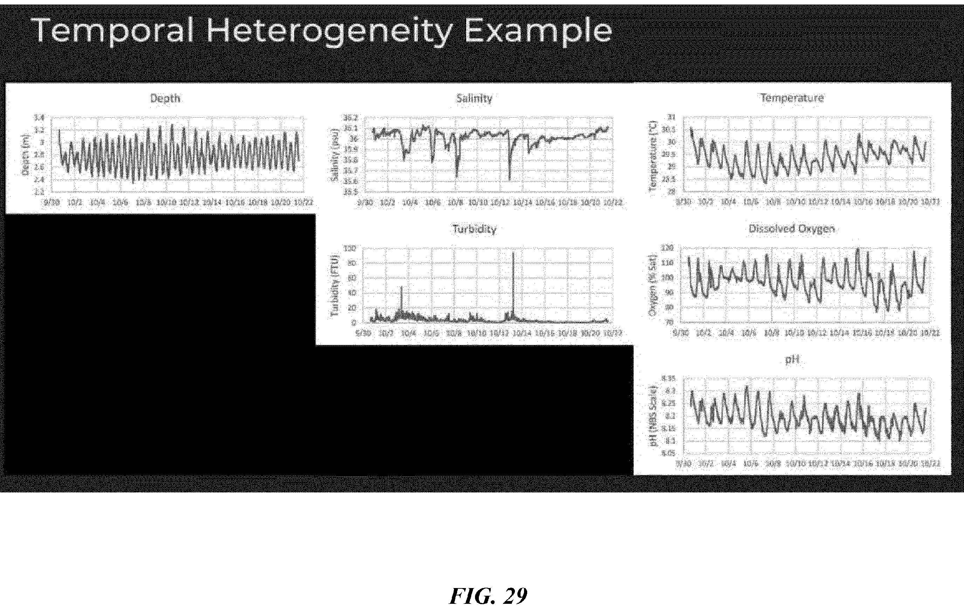

In some cases, the methods disclosed herein may comprise assessing or evaluating temporal heterogeneity by measuring depth, temperature, salinity, pH, turbidity, chlorophyll concentrations, and/or dissolved oxygen concentrations over time. **FIG. 29** shows plots of temporal heterogeneity of various characteristics for a target site, including depth, salinity, turbidity, temperature, dissolved, oxygen, and pH. In some cases, the target site for olivine distribution and dissolution may be chosen based on temporal heterogeneity relative to other candidate or baseline/reference sites.

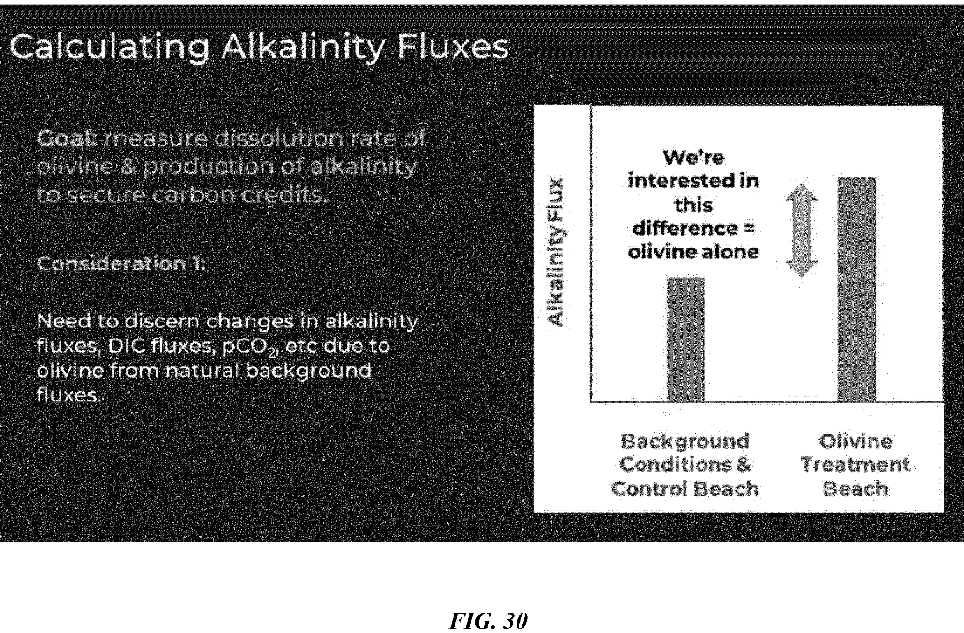

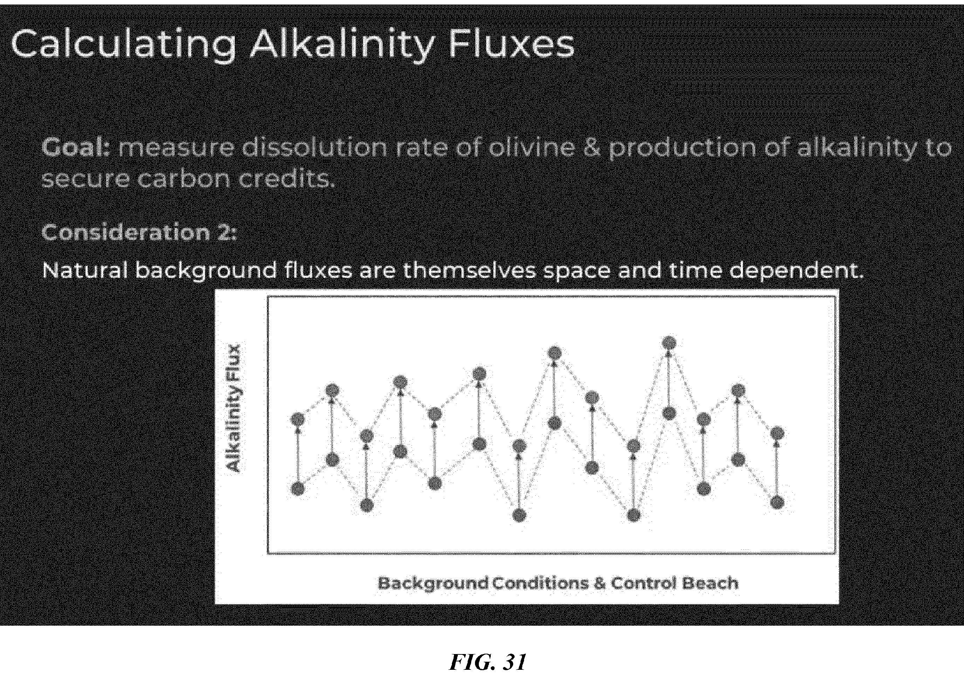

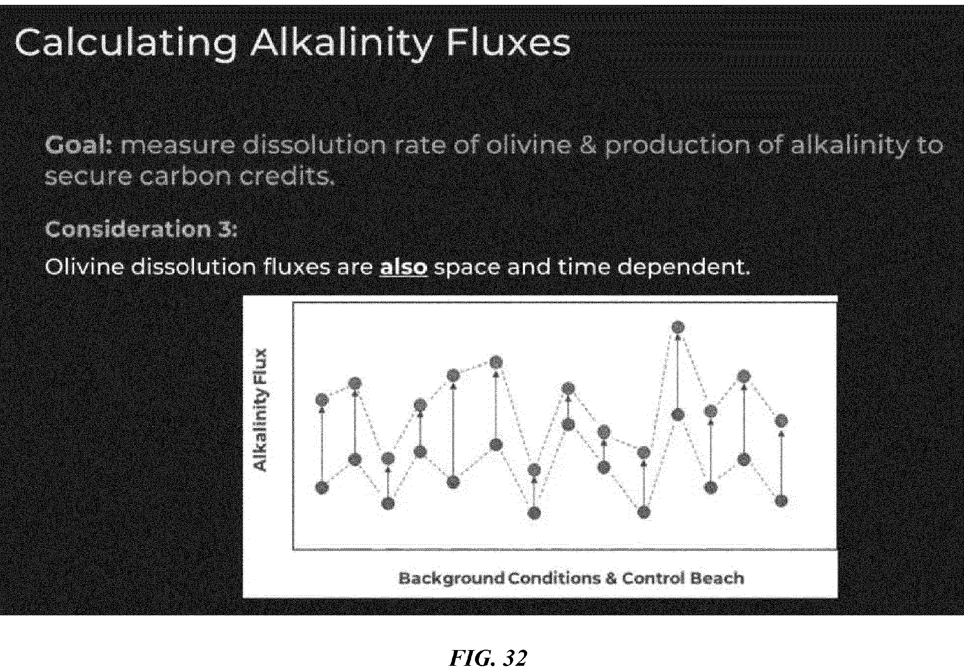

As shown in **FIGs. 30 - 32**, in some cases, the methods disclosed herein may comprise calculating alkalinity fluxes and measuring dissolution rates of olivine to secure carbon credits. Alkalinity fluxes induced by olivine may be determined by discerning changes in alkalinity fluxes due to olivine from other natural background fluxes. In some cases, other fluxes attributable to olivine, such as dissolved inorganic carbon (DIC) fluxes and fluxes of pCO2, may be determined based on a comparison of measured fluxes to natural background fluxes. The fluxes attributable to olivine dissolution and/or the natural background fluxes may be space and time dependent. Such variations over space and time may be simulated, modeled, tracked, and/or measured to ensure accurate calculation of fluxes attributable to olivine, as opposed to natural background fluxes.

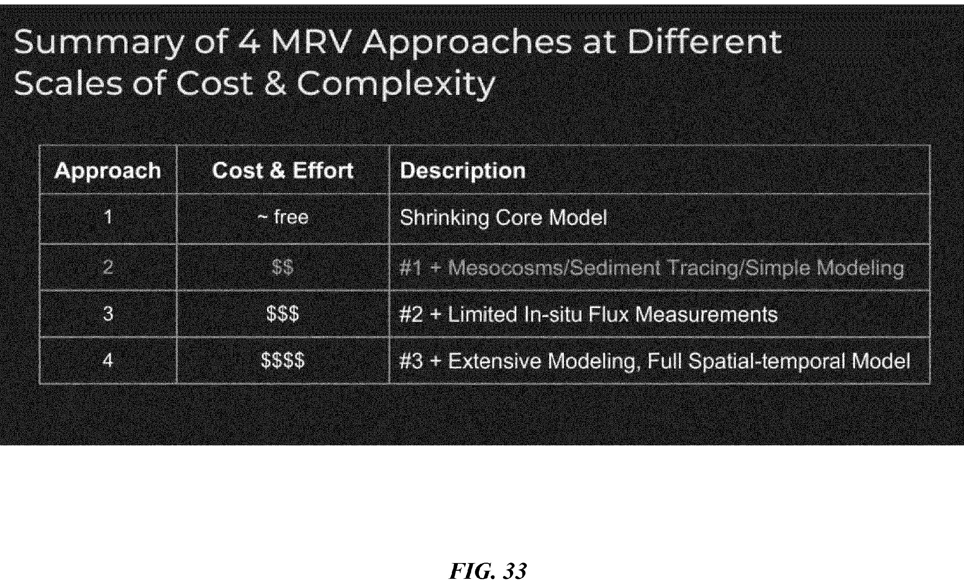

In another aspect, the present disclosure provides various methods for accounting for heterogeneity for MRV. **FIG. 33** shows a summary of the MRV approaches described below at different scales of cost and complexity.

**Approach 1** - In some cases, the methods may involve using the shrinking core model described elsewhere herein for an approximate rate of dissolution. The dissolution rate and CO₂/olivine ratio may be assumed or approximated.

**Approach 2** - Alternatively, mesocosm experiments may be utilized to determine regional olivine dissolution rates. In cases where olivine is deployed in a target site (e.g., a mesocosm or the natural environment), olivine tracers may be used to track olivine dissolution in order to model and refine in situ weathering rates. The present disclosure provides systems and methods to track the movement of olivine grains in a target site. The systems and methods of the present disclosure may be implemented to track olivine transport in a target site based on or using, for example, trace metal content, fluorescent dyes, and/or olivine-specific spectral properties. This may be a relatively inexpensive process that can be set up prior to obtaining regulatory permits. Further, this approach may be initially tested using a sealed, recirculating design that is configured to maintain and control spatiotemporal heterogeneity and further allow for temperature and/or lighting control.

**Approach 3** - In some cases, the mesocosm-based approaches described herein may be augmented with field data (e.g., in situ data obtained manually or automatically using one or more sensors). The data obtained using the one or more sensors (which one or more sensors may comprise any of the sensors described herein or any other sensors for obtaining any of the measurements referred to herein) may provide additional information to better quantify and track spatial and/or temporal variations in environmental conditions or parameters relating to olivine dissolution and coastal carbon capture.

**Approach 4** - In some cases, a numerical model may be constructed that allows for accurate simulation of spatial and temporal variations in background fluxes and olivine dissolution rates. This can help to correct for time and space dependent variations, and allow for the use of sparse observations or other information or data at discrete points (e.g., points in time and/or points in space) to calibrate, verify, and validate the model. The model may be built and implemented for one or more a priori simulations.

In cases where olivine is deployed in the natural environment, olivine tracers may be used to track olivine dissolution in order to estimate, predict, model, and refine in situ weathering rates. The present disclosure provides systems and methods for tracking the movement or transport of olivine grains in the natural environment (e.g., in a target site in which the olivine is provided or introduced). The systems and methods of the present disclosure may be implemented to track olivine transport based on or using, for example, trace metal content, fluorescent dyes, and/or olivine-specific spectral properties. The trace metal content, the movement or dispersion of fluorescent dyes, and/or the olivine-specific spectral properties may be detected and tracked using any of the sensors described herein.

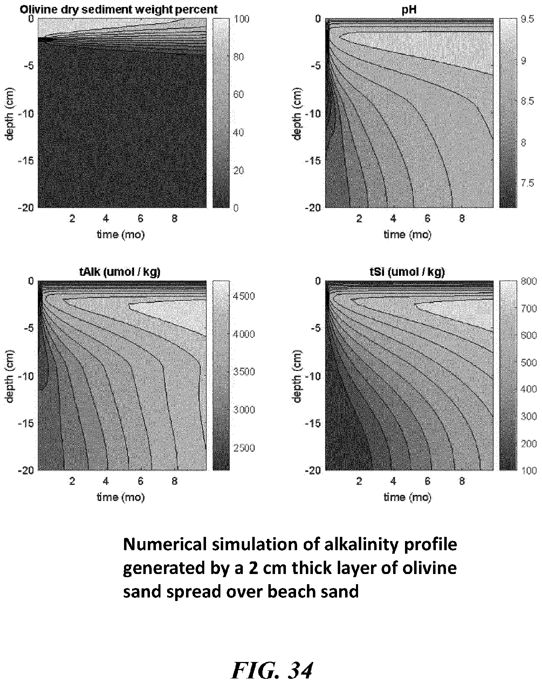

**FIG. 34** illustrates various examples of reaction-transport modeling studies that may be used to simulate sediment porewater profiles, solid-phase chemistry, and benthic fluxes. The simulations and studies may be tailored to project requirements, and can take into account secondary clay mineralogy and Cr and Ni speciation and cycling. The plots shown in **FIG. 34** illustrate an exemplary numerical simulation of alkalinity profile and the effects of spreading a 2 centimeter (cm) thick layer of pure olivine sand on a beach sediment. In some cases, the alkalinity flux across the sediment-water interface may be monitored by (1) constructing biogeochemical models of olivine dissolution in sediments, (2) validating model performance using field measurements of sediment-water alkalinity flux (and other carbonate parameters), and (3) calculating CO2 capture, storage, or sequestration (by weight or volume) based on the thermodynamics of air-sea CO2 exchange.

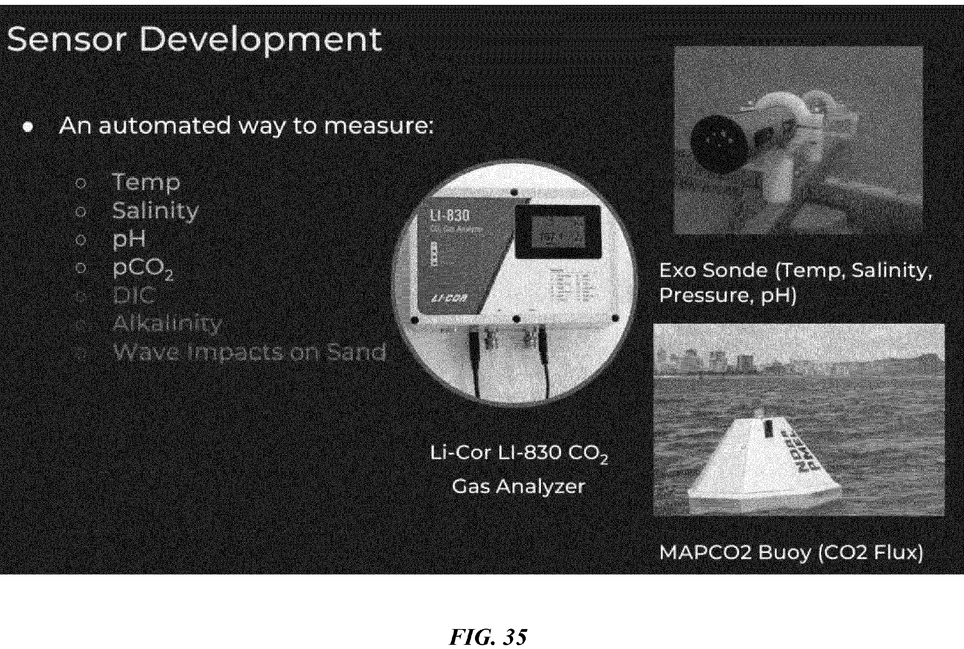

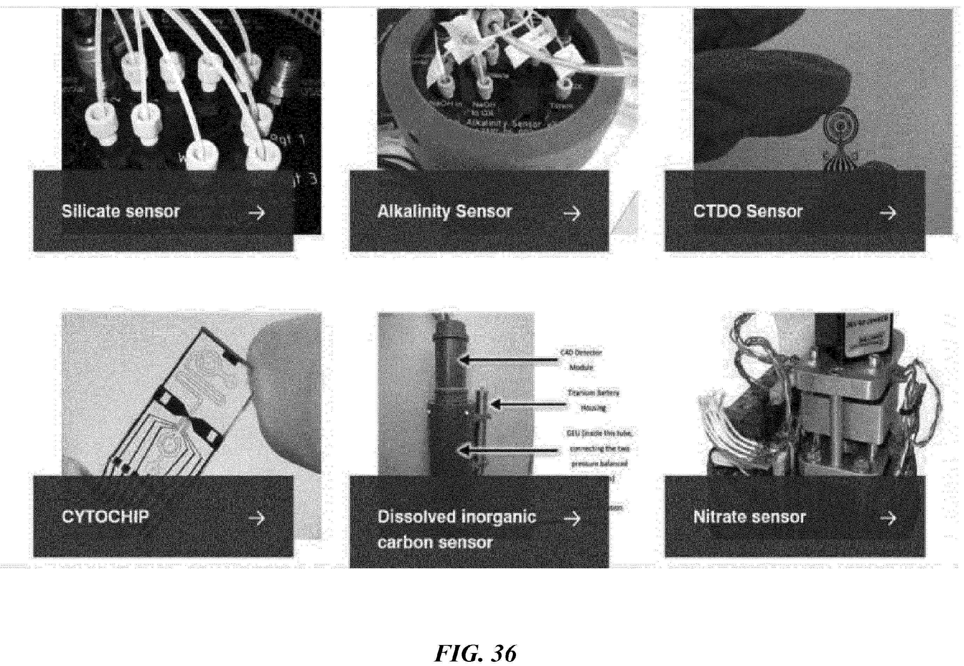

The systems and methods disclosed herein may be implemented using one or more sensors capable of measuring at optimal spatial and temporal resolutions. The sensors may obviate the need to use traditional, manual geochemical methods, which require labor intensive field sampling and returning of samples to labs for analysis and can involve significant costs. **FIG. 35** shows various examples of sensors that can be used for MRV, including sensors configured to measure temperature, salinity, pH, pCO2, DIC, alkalinity, and/or wave impacts on sand. In some cases, a solid state sensor for simultaneous measurement of total alkalinity and pH of seawater (including seawater that does or does not comprise olivine) may be used. In some embodiments, a plurality of sensors for MRV may be deployed on a single system. In other embodiments, a plurality of sensors for MRV may be deployed on multiple separate systems. Additional examples of sensors are shown in **FIG. 36**, including silicate sensors, alkalinity sensors, CTDO sensors, Cytochips, dissolved inorganic carbon sensors, and nitrate sensors. In some cases, a ²⁹Si isotopic tracer can be used to track olivine dissolution and simultaneous precipitation of secondary clays and carbonate phases. This can allow separation of total and net silicate dissolution.

In some cases, the impacts of physical wave-driven weathering on beach sand and olivine dissolution may be measured or simulated. In some cases, this may involve measuring and analyzing 2-phase (i.e., sand-water) flow in the benthic boundary layer of a breaking wave. This may be modeled numerically or physically (e.g., using wave tanks). In some cases, specialized sensors for measuring turbulent energy dissipation (e.g., in a lab or directly on a beach) may be utilized to measure impacts of physical wave-driven weathering.

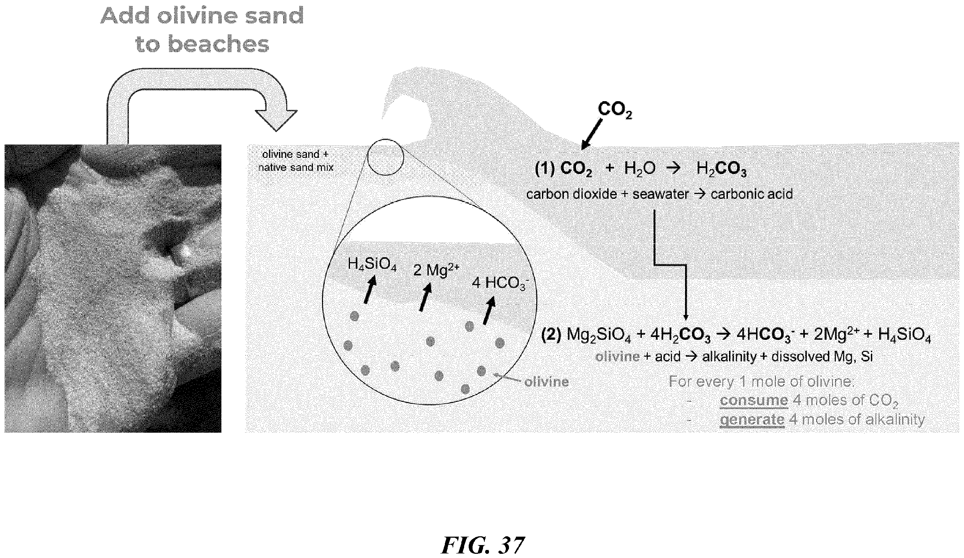

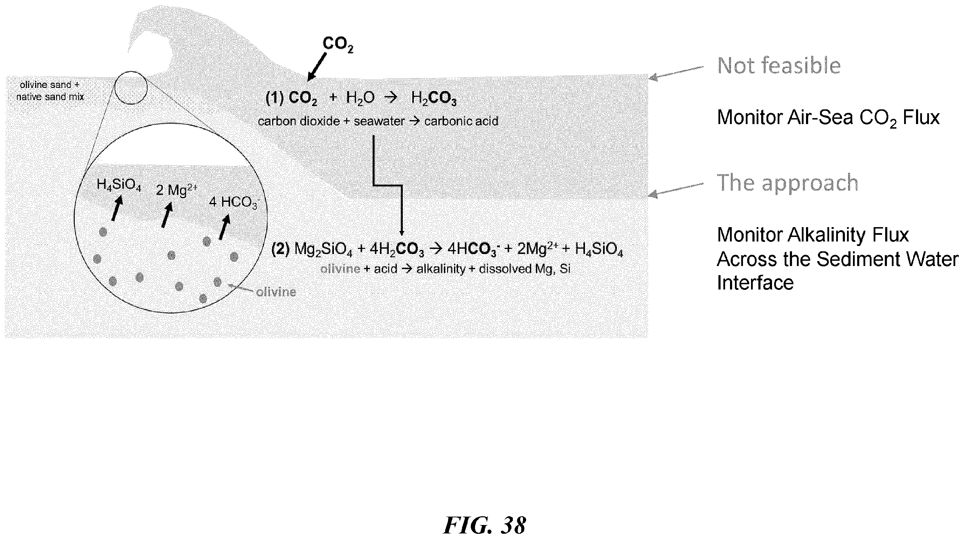

**FIG. 38** illustrates an approach to measuring CO2 removal that is enabled by way of the reactions shown in **FIG. 37**. In some cases, it may not be feasible to monitor the air to sea CO2 flux. Measuring air-sea CO2 fluxes can be challenging because the olivine dissolution signal is strongly muted by dilution with open ocean seawater. For instance, a 1 cm thick layer of olivine dissolving in 5 meters of water in a bay with a 4-hour residence time implies a ~ 1.3 µM increase in water column alkalinity. Measuring net CO2 flux can also be complicated by extreme seasonal and diurnal variations in ΔPCO2ₐᵢᵣ₋ₛₑₐ due to changes in local photosynthesis and respiration. Accordingly, in some embodiments, the systems and methods disclosed herein may be implemented to monitor alkalinity flux across the sediment-water interface.

In some cases, the models described herein (which models may be generated for specific sites) may undergo validation. Such validation may involve determining olivine abundance, spatial distribution, and dissolution rates at one or more reference points in space or time. In some cases, the reference points may be arranged in a grid pattern. Validation may further involve crossvalidating measured and modeled alkalinity fluxes and olivine dissolution at reference points, and calculating alkalinity flux for a region or a project based on (i) a known mass or volume of olivine deployed and (ii) validated site-specific modeling. The models may be validated using one or more sensor measurements as described elsewhere herein. Once validated, the models may be used to determine or predict olivine dissolution, reaction fluxes, and/or carbon sequestration. In some cases, the models may be updated or refined based on additional sensor measurements captured over a period of time. In some cases, the models may be revalidated based on additional sensor measurements.

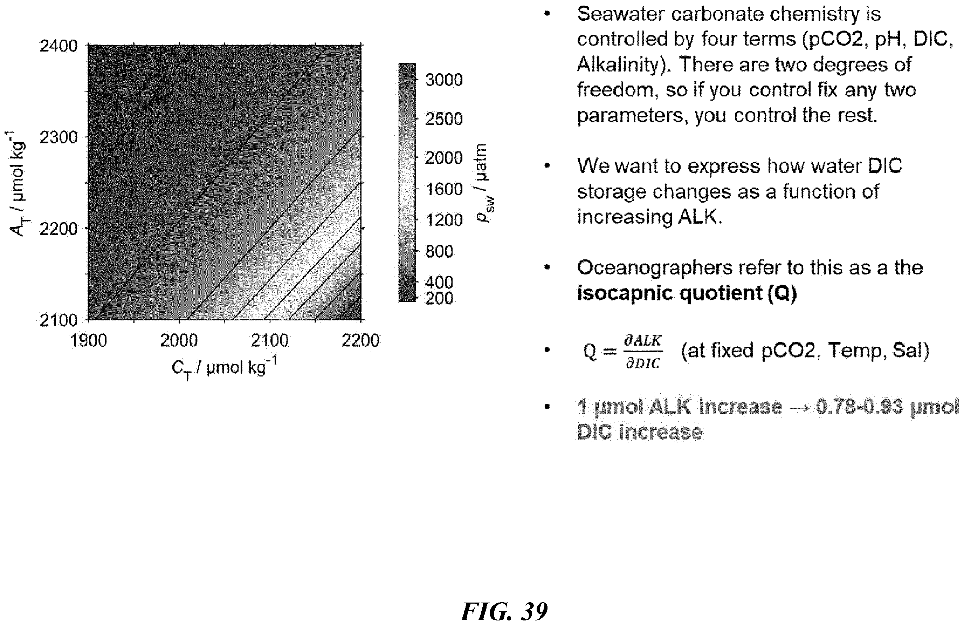

**FIG. 39** illustrates an approach to calculating CO2 sequestration from alkalinity flux based on an expression of how water DIC storage changes as a function of increasing alkalinity. Seawater carbonate chemistry may be controlled by pCO2, pH, DIC, and alkalinity. An isocapnic quotient may be computed based on a change in alkalinity relative to a change in DIC, at fixed conditions for pCO2, temperature, and salinity. In some cases, a 1 micromol increase in alkalinity may yield a 0.78-0.93 micromol DIC increase, which means that for every one ton of forsterite provided to a target site, 0.97 to 1.16 tons of CO2 can be stored as marine DIC.

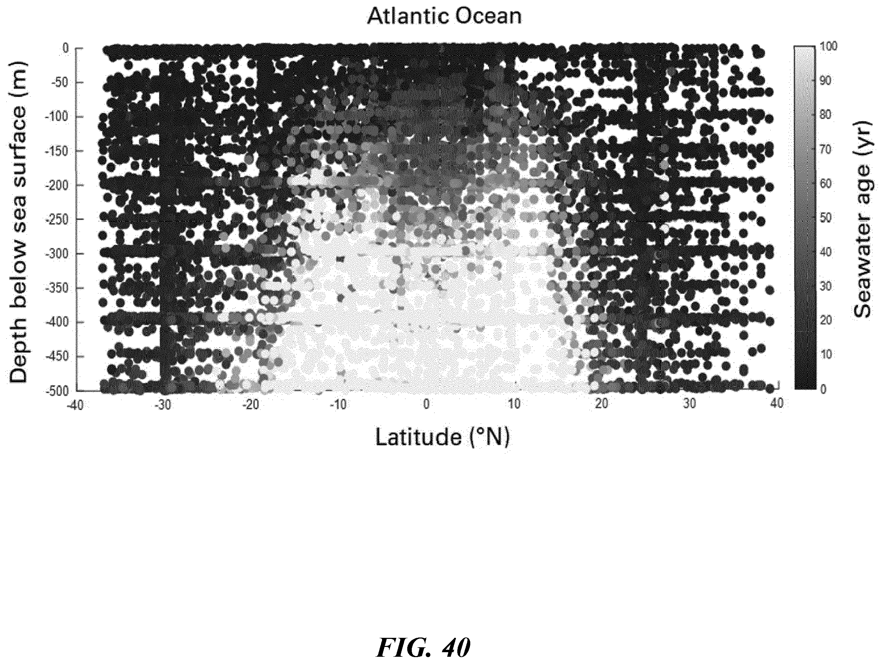

**FIG. 40** illustrates a plot showing seawater age and the depth below sea water level of the Atlantic Ocean as a function of latitude (degrees North). CO2 capture via ocean alkalinization requires that the enhanced alkalinity water mass achieves equilibrium with atmospheric pCO2. The precise timescale for pCO2 equilibrium (~ 4 months) and DIC adjustment (~4 years) depends on the Revelle factor (Jones et al. 2014). The time scale for air-sea equilibration is considerably shorter than the time scale for olivine dissolution (~ 10 - 100 year) and approximately similar to the transit time distribution of mixed layer and subtropical mode water (- 10 - 40 years). This means that the surface DIC reservoir can achieve quasi-steady equilibrium throughout equatorial and subtropical regions most suitable for coastal enhanced weather.

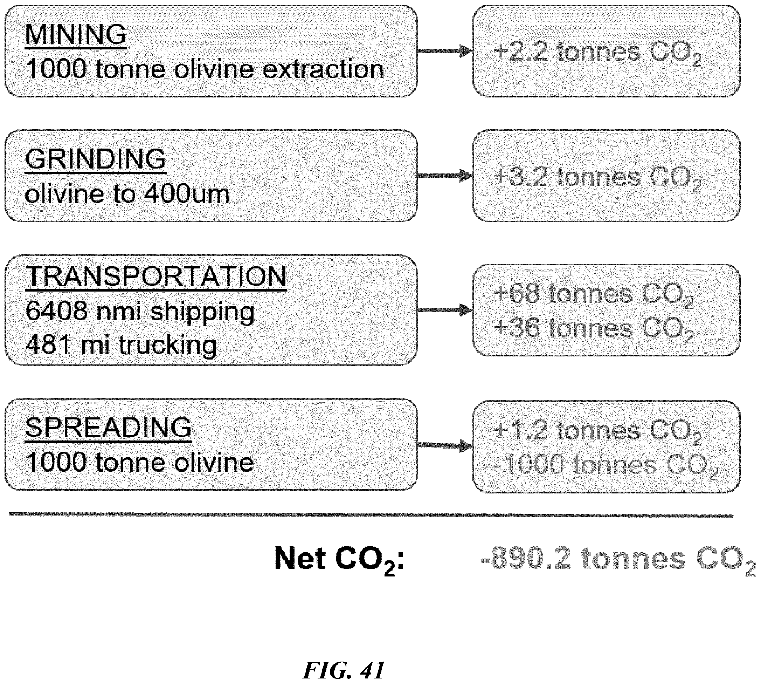

**FIG. 41** illustrates an exemplary CEW life cycle analysis. The life cycle may comprise mining and/or extracting olivine, grinding the olivine to approximately 400 micrometer grain sizes (or less), transporting the olivine to one or more target sites, and spreading the olivine at the target sites. In some cases, the life cycle may be at least about 89% efficient, and can enable sequestration of at least about 5 to 20 times more CO2 than the amount of CO2 emitted during mining, grinding, transportation, and/or spreading of olivine.

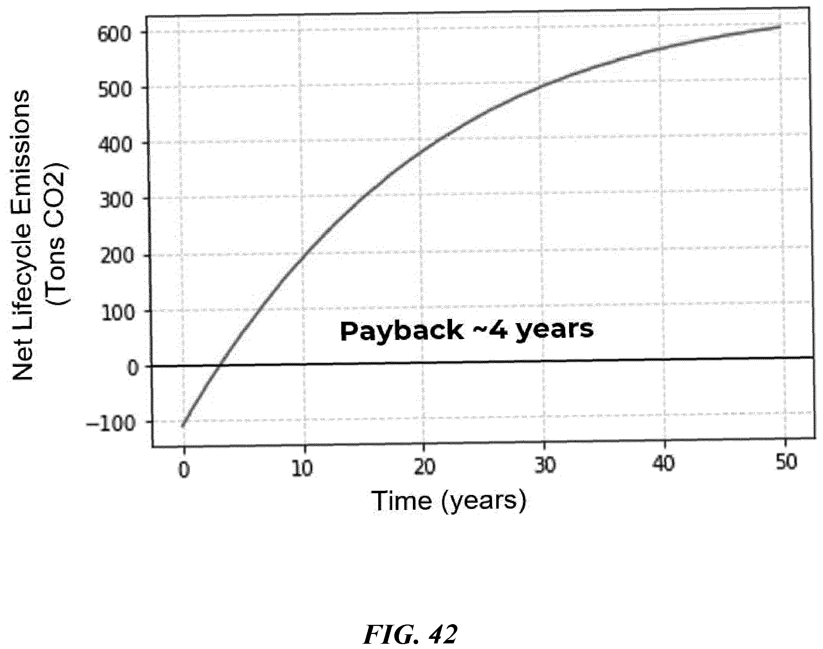

**FIG. 42** illustrates an example of a carbon payback period that can be realized using the methods and systems disclosed herein. In some cases, the break even payback period may occur at approximately 4 years, assuming the following:
Grainsize: LE45 (d50: 365 µm)
Temp: 25 °C
pH: 8
Embedded emissions: 110 tons CO2 / 1000 tons olivine
Absorption efficiency: 3 moles ALK / mole olivine
Dissolution constant: Log(r) = - 8.75 (can vary depending on pH/temp)

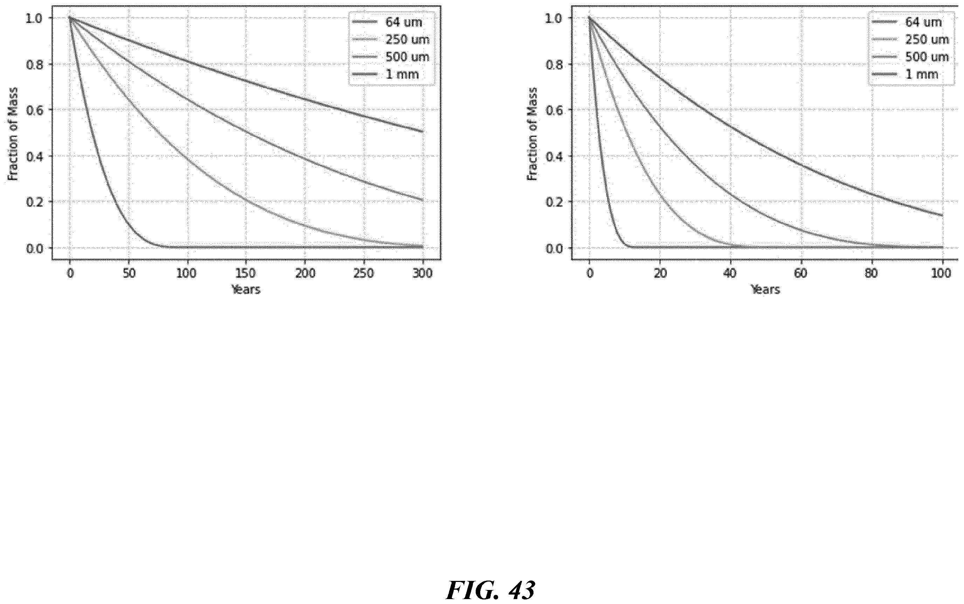

**FIG. 43** illustrates various plots showing exemplary olivine dissolution kinetics for olivine particles having different grain sizes. Dissolution time may decrease as olivine particle sizes decrease. The particle dissolution characteristics may be predicted or simulated using a shrinking core model and one or more laws, equations, or principles governing olivine dissolution rates. In some cases, the shrinking core model may be modified to permit the simulation of dissolution for realistic, commercially-available grain size distributions. The models described herein may be generated by modifying initial diagenesis models to incorporate olivine dissolution kinetics that correspond to the shrinking core model. The models may include and/or account for full carbonate chemistry (e.g., DIC, ALK, pCO2, pH, carbonate precipitation/dissolution) as well as biogeochemical processes (including aerobic and anaerobic respiration, formation of Fe/Mn oxides, pyrite, sulfide oxidation, etc).

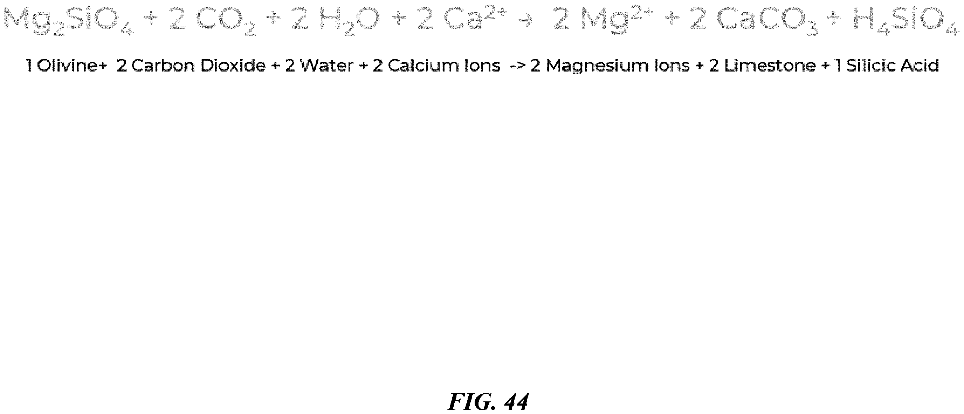

In some instances, secondary carbonate precipitation as shown in **FIG. 44** can impact CO2 capture efficiency. Secondary carbonate precipitation may change the reactions described herein such that for every 1 mol of olivine introduced to a target environment, 2 moles of CO2 are consumed and 0 moles of alkalinity are generated. This can result in a 50% reduction in CO2 capture efficiency. The systems and methods disclosed herein may be implemented to minimize or reduce secondary carbonate precipitation. The rate of carbonate precipitation can be strongly controlled by local variations in geochemistry and biogenic calcification. In some cases, alkalinity export can be the dominant process occurring in sediments, and once mixed into the water column, consumption of alkalinity can be low given the long residence time of DIC and ALK in seawater.

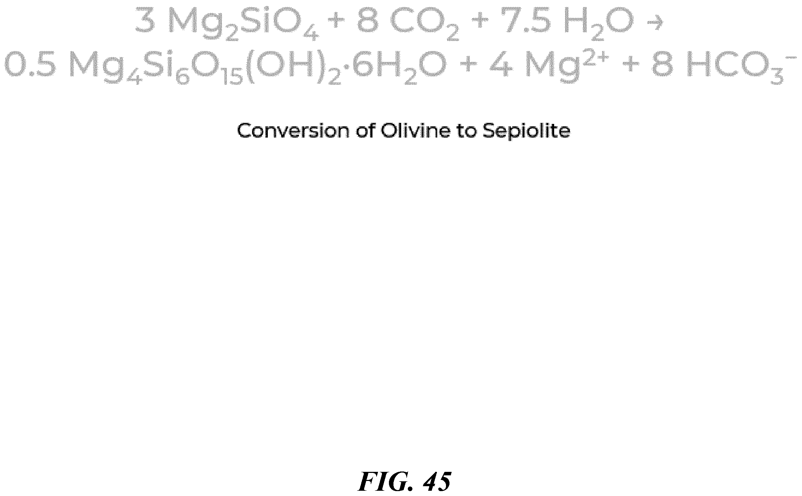

In some instances, secondary clay formation as shown in **FIG. 45** can impact CO2 capture efficiency. Secondary clay formation may change the reactions described herein such that for every 1 mol of olivine, 8/3 moles of CO2 are consumed and 8/3 moles of alkalinity are generated. This can result in a 33% reduction in CO2 capture efficiency. The systems and methods disclosed herein may be implemented to minimize or reduce such secondary clay formation.

In a related aspect, the present disclosure provides systems and methods for simulating a target site or environment. This can allow the study of olivine dissolution rates in a controlled environment, intermediate between lab and field conditions. Such systems and methods may be implemented to determine and/or monitor olivine dissolution rates, porewater geochemistry, or benthic flux measurements, study impacts of temperature on olivine dissolution and respiration, track impacts on seawater pH and other water chemistry, monitor ecological responses and air-sea water gas CO2 fluxes, track the fate, transport, and bioaccumulation of Cr and Ni, track the formation of secondary weathering products (e.g., Chrysotile, Carbonates, Clays), and monitor the impacts of olivine-based CO2 sequestration processes on benthic invertebrates/meiofauna.

In some embodiments, the MRV methods and protocols disclosed herein may be adapted or adjusted for different types of olivine or for different target sites for olivine distribution. In such cases, the models used to predict or determine olivine dissolution, reaction fluxes, and/or carbon sequestration may be adjusted based on characteristics of the particular olivine/sand mixture used, the target site, and/or one or more sensor readings or measurements obtained in situ.

### Carbon Removal Prediction

[6] In another aspect, the present disclosure provides systems and methods for predicting rates of carbon removal at specific sites. Such predictions may be based on, for example, data corresponding to measurement of changes in alkalinity, pCO2, pH, and/or water temperature. The predictions may be generated using algorithms and software interpreting such data. The algorithms and software may be implemented by way of one or more models that can be generated based on the MRV data obtained using various sensors as described elsewhere herein.

In some embodiments, the predicted rates of carbon removal may be used to further optimize the carbon capture process. For instance, the predicted rates may be used to optimize the properties of the olivine sand, the preparation of the olivine sand, and/or the operation or the configuration of the reactor.

### Shipping and Deployment of Olivine

[7] In another aspect, the present disclosure provides systems and methods for shipping and deploying olivine. In some cases, the shipping and deployment of olivine may be performed using a device for distributing carbon negative sand in shallow marine environments. The device may be, for example, a spreading device for spreading the olivine in a desired or optimal manner at a target site (e.g., a beach of interest).

In some cases, the methods of the present disclosure may comprise determining whether one or more dredging modifications are needed in order to optimally distribute the carbon negative sand. Such determination may be based on one or more sensor readings and/or information about the target site in which the carbon negative sand is to be distributed.

In some cases, the olivine may be processed before shipping to facilitate shipping and distribution of the olivine. For instance, the olivine may be modified to enable transportation of ultra-fine material (e.g., for reduction of liquefaction or capsizing risk.

In some embodiments, various types and forms of maritime emissions reduction technology (e.g., an on-board olivine reactor driven by ship engine using ballast water) may be implemented. In some cases, bulk carrier direct deployment technology and related modifications (e.g., open sea distribution for in situ CEW) may be utilized. In some cases, grain washing and liquid alkalinity systems may be used for pre-deployment reduction of turbidity. In some cases, global logistics software may be used to mobilize idle maritime assets for carbon removal projects.

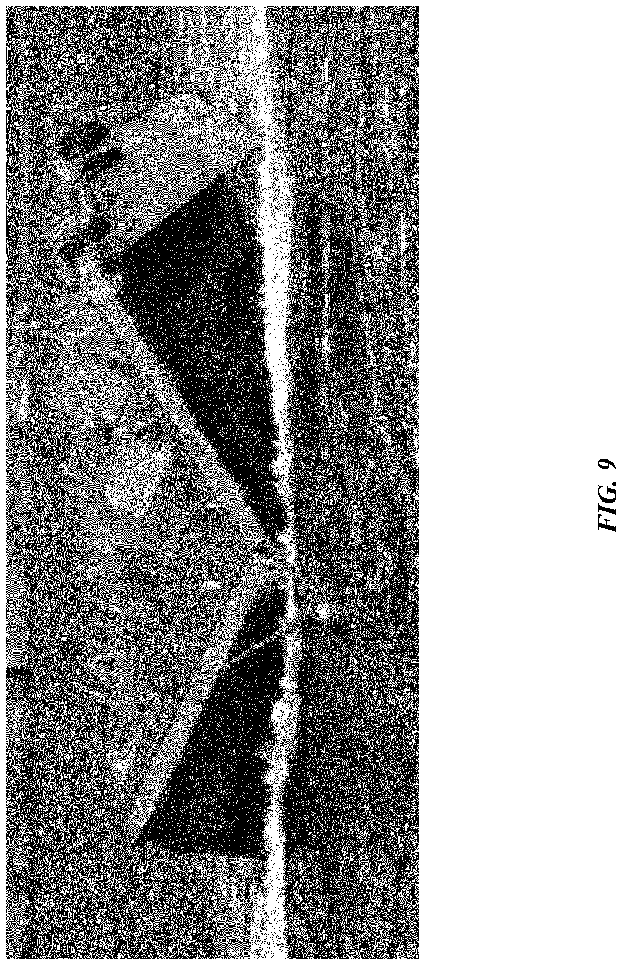

**FIG. 9** schematically illustrates an example of a system that may be used to deploy olivine for coastal nourishment. The system may comprise, for example, a split hulled hopper barge. In some cases, olivine may be mined and milled into carbon negative sand. Thereafter, the olivine may be loaded and shipped on dry bulk carriers to designated ports. The olivine may then be loaded into one or more split hulled hopper barges. In some embodiments, the barges may be linked together and towed to designated deployment sites. Once the barges are positioned at or near the designated deployment sites, the bottom of the barges may be configured to open in order to deploy the olivine. In some cases, sediment transport modeling may be used to predict where the olivine will move and/or in what directions the olivine will disperse.

In a further aspect, the present disclosure provides a method and process for conducting carbon neutral and carbon negative dredging operations. The method and process of conducting dredging operations may be either net neutral or net negative with respect to atmospheric greenhouse gas emissions. To achieve this, dredged sediment may be mixed with one or more types of carbon negative sand, as described elsewhere herein. The methods and processes described herein may be adapted for any type of construction activity, including land-based construction activities.

The dissolution of carbon negative sand may effect the conversion of gaseous and dissolved carbon dioxide into dissolved bicarbonate and carbonate ions, thereby increasing the capacity of fresh and salt water to store additional carbon in a dissolved state. When present as dissolved bicarbonate and carbonate ions, rather than carbon dioxide, these compounds may no longer contribute to climate change and can act to counter the effect of ocean acidification.

In order to reduce, neutralize, or negate carbon dioxide emissions which occur during the combustion of fossil fuels during conventional dredging operations, a predefined ratio of carbon negative sand may be mixed with the dredging spoils in order to [1] result in the net capture of a specified amount of carbon dioxide and [2] ensure that the dredged sediments meet engineering and regulatory requirements for disposal or beneficial reuse. Blends consisting of 1-50% reactive carbon negative component may be most common, but in other variations, higher or lower fractions of the carbon negative component may be used.

In order to mix carbon negative sand with dredged materials, several different approaches may be used depending on the type of dredging operation and intended fate of the dredged material. During cutterhead dredging operations, pump-out hopper dredging, and other approaches where dredged material is conveyed via a hydrologic slurry, the carbon negative sand may be directly mixed with the dredge spoils by injecting the carbon negative sand into the slurry pipeline. When using trailing suction hopper dredges, carbon negative sand may be mixed within a sediment hopper or barge prior to discharge. As an alternative, carbon negative sand may be directly placed at the sediment destination in the specified quantity and allowed to mix with the dredge spoils via the action of wind, waves, tides, and currents.

Whereas traditional dredging operations consume large quantities of fuel fuels and emit correspondingly large quantities of greenhouse gasses, the method and process described here may allow dredging operations to be conducted in a way that is carbon neutral or even carbon negative over the lifetime of the project. This method and process provides significant advantages, allowing dredging operators to (i) mitigate carbon dioxide emissions on behalf of themselves or their clients, (ii) avoid emissions caps and taxes, and (iii) operate in jurisdictions which impose voluntary or involuntary restrictions on greenhouse gas emissions.

### Carbon-removing marine landfill cap

In another aspect, the present disclosure provides carbon-removing marine landfill caps, processes for making the same, and processes for quantifying carbon capture from carbon-removing marine landfill caps. Marine landfills may serve as long-term, seafloor placement areas for unwanted construction debris, dredging materials, and toxic pollutants. Currently, marine landfills are often "capped" with non-toxic clay or sand or other similar materials to prevent contamination of overlying seawater and allow marine life to thrive in previously environmentally damaged areas. By using carbon-removing sand wholly, or as a constituent of, marine landfill caps, these sites can serve as climate change mitigation locales and further encourage the regeneration of environmentally damaged areas through the production of bicarbonate (alkalinity) which offsets ocean acidification and is beneficial for marine life.

The process of making and deploying carbon-removing marine landfill cap differs from traditional marine landfill cap because 1) the depth (thickness) of carbon-removing cap sand 2) grain size distribution of carbon-removing cap sand, and 3) possible blend of carbon-removing cap and standard cap must be optimized to 1) prevent release of toxic pollutants and similar while allowing for sufficient permeability so that the carbon-removing sand will capture carbon over time and in particular not saturate sand porewaters such that the carbon removal reaction slows or stops. To achieve these objectives, a carbon-removing marine landfill cap may be deployed wholly as a cap, applied as a single mixture of carbon-removing cap to regular cap sand, or applied in layers where the percentage of carbon-removing cap sand to regular cap sand changes with depth, or the grain size distribution of carbon-removing cap and/or standard cap changes with depth. In all circumstances the carbon capture rates may vary with depth. In some cases, the carbon capture rate may be determined based on analytical measurements and sensor measurements from a particular site, fit to a global model. In some embodiments, the appropriate deployment technique described above may be a function of local wave climate, ecology (bioturbation), seawater temperature, landfill material, and an LCA (life cycle analysis) as the CO₂ emitted to deploy the carbon-removing cap must be accounted for to insure the cap is, indeed, carbon-removing.

### Green Hydrogen

[8] In another aspect, the present disclosure provides systems and methods for generating and using green hydrogen. As used herein, the term "green hydrogen" may refer to hydrogen that is produced from renewable energy sources and/or low-carbon power sources. The green hydrogen may be used as fuel for fuel cells or internal combustion engines. In some cases, the green hydrogen may be generated based on one or more products or reactants used to drive the CO2 capture and sequestration reactions/methods described elsewhere herein.

### Computer Systems

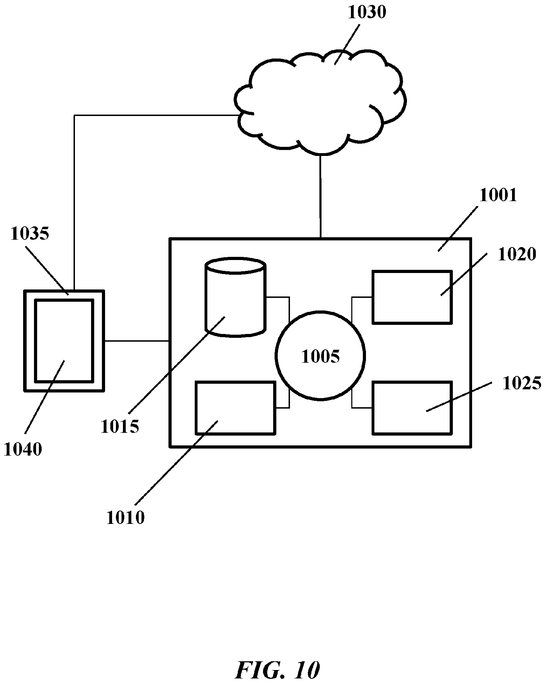

In an aspect, the present disclosure provides computer systems that are programmed or otherwise configured to implement methods of the disclosure, e.g., any of the subject methods for processing and distributing olivine. **FIG. 10** shows a computer system 1001 that is programmed or otherwise configured to implement a method for olivine processing and distribution. The computer system 1001 may be configured to, for example, (i) identify a target site, (ii) optimize one or more procedures for processing olivine to yield favorable properties or characteristics for the olivine, based on the target site identified, and (iii) coordinate transportation of the olivine to the target site. The computer system 1001 can be an electronic device of a user or a computer system that is remotely located with respect to the electronic device. The electronic device can be a mobile electronic device.

The computer system 1001 may include a central processing unit (CPU, also "processor" and "computer processor" herein) 1005, which can be a single core or multi core processor, or a plurality of processors for parallel processing. The computer system 1001 also includes memory or memory location 1010 (e.g., random-access memory, read-only memory, flash memory), electronic storage unit 1015 (e.g., hard disk), communication interface 1020 (e.g., network adapter) for communicating with one or more other systems, and peripheral devices 1025, such as cache, other memory, data storage and/or electronic display adapters. The memory 1010, storage unit 1015, interface 1020 and peripheral devices 1025 are in communication with the CPU 1005 through a communication bus (solid lines), such as a motherboard. The storage unit 1015 can be a data storage unit (or data repository) for storing data. The computer system 1001 can be operatively coupled to a computer network ("network") 1030 with the aid of the communication interface 1020. The network 1030 can be the Internet, an internet and/or extranet, or an intranet and/or extranet that is in communication with the Internet. The network 1030 in some cases is a telecommunication and/or data network. The network 1030 can include one or more computer servers, which can enable distributed computing, such as cloud computing. The network 1030, in some cases with the aid of the computer system 1001, can implement a peer-to-peer network, which may enable devices coupled to the computer system 1001 to behave as a client or a server.

The CPU 1005 can execute a sequence of machine-readable instructions, which can be embodied in a program or software. The instructions may be stored in a memory location, such as the memory 1010. The instructions can be directed to the CPU 1005, which can subsequently program or otherwise configure the CPU 1005 to implement methods of the present disclosure. Examples of operations performed by the CPU 1005 can include fetch, decode, execute, and writeback.

The CPU 1005 can be part of a circuit, such as an integrated circuit. One or more other components of the system 1001 can be included in the circuit. In some cases, the circuit is an application specific integrated circuit (ASIC).

The storage unit 1015 can store files, such as drivers, libraries and saved programs. The storage unit 1015 can store user data, e.g., user preferences and user programs. The computer system 1001 in some cases can include one or more additional data storage units that are located external to the computer system 1001 (e.g., on a remote server that is in communication with the computer system 1001 through an intranet or the Internet).

The computer system 1001 can communicate with one or more remote computer systems through the network 1030. For instance, the computer system 1001 can communicate with a remote computer system of a user (e.g., an end user performing or monitoring the processing and/or the transportation or distribution of the olivine). Examples of remote computer systems include personal computers (e.g., portable PC), slate or tablet PC's (e.g., Apple^{®} iPad, Samsung^{®} Tab), telephones, Smart phones (e.g., Apple^{®} iPhone, Android-enabled device, Blackberry^{®}), or personal digital assistants. The user can access the computer system 1001 via the network 1030.

Methods as described herein can be implemented by way of machine (e.g., computer processor) executable code stored on an electronic storage location of the computer system 1001, such as, for example, on the memory 1010 or electronic storage unit 1015. The machine executable or machine readable code can be provided in the form of software. During use, the code can be executed by the processor 1005. In some cases, the code can be retrieved from the storage unit 1015 and stored on the memory 1010 for ready access by the processor 1005. In some situations, the electronic storage unit 1015 can be precluded, and machine-executable instructions are stored on memory 1010.

The code can be pre-compiled and configured for use with a machine having a processor adapted to execute the code, or can be compiled during runtime. The code can be supplied in a programming language that can be selected to enable the code to execute in a pre-compiled or ascompiled fashion.

Aspects of the systems and methods provided herein, such as the computer system 1001, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media including, for example, optical or magnetic disks, or any storage devices in any computer(s) or the like, may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 1001 can include or be in communication with an electronic display 1035 that comprises a user interface (UI) 1040 for providing, for example, a portal for a user to monitor the processing and/or the transportation or distribution of the olivine. The portal may be provided through an application programming interface (API). A user or entity can also interact with various elements in the portal via the UI. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 1005. For example, the algorithm may be configured to identify a target site and optimize a procedure for processing the olivine based on the properties or the characteristics of the target site.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

## Claims

1. A system for processing olivine, comprising:
a reactor for accelerating olivine weathering, wherein the reactor comprises one or more chambers for housing one or more microbes, biological mediators, or enzymatic accelerants to facilitate said olivine weathering.

2. The system of claim 1, wherein the reactor is configured to adjust one or more operational parameters for processing olivine based on one or more sensors configured to measure carbon capture facilitated by said olivine weathering.

3. The system of claim 1 or 2, wherein the olivine comprises pelletized olivine aggregates formed from olivine fines.

4. The system of claim 3, wherein the olivine is prepared using [1] use of a binding agent, [2] via encapsulation of the olivine in one or more select materials or [3] via thermal fusion or physical compression of smaller olivine grains into larger aggregates.

5. The system of claim 3 or 4, wherein the pellets are designed to either [i] remain substantially intact while maintaining improved surface area via internal porosity and permeability or [ii] degrade in a controlled fashion to release fine olivine fines to the environment in a prolonged controlled fashion.

6. The system of any one of claims 3 to 5, wherein the preparation of the pellets allows olivine dissolution to occur substantially faster than would occur for equivalently sized solid pure olivine grains, improving the process of carbon capture, while minimizing the aesthetic, health, and ecological harm and challenges posed by working with and utilizing olivine mineral fines.

7. A method, comprising:
(a) generating one or more models for determining or predicting olivine dissolution, carbon sequestration, or one or more reaction fluxes associated with a dissolution of olivine in a target environment;
(b) obtaining one or more sensor measurements at the target environment;
(c) validating and/or updating the one or more models based on the one or more sensor measurements; and
(d) using the one or more validated and/or updated models to determine or predict (i) olivine dissolution, (ii) carbon sequestration, and (iii) the one or more reaction fluxes in the target environment.

8. The method of claim 7, wherein the target environment comprises a coastal or aquatic environment.

9. The method of claim 7 or 8, further comprising providing olivine to the target environment.

10. The method of claim 9, wherein the olivine is provided as part of a mixture comprising (i) the olivine and (ii) local beach sand at the target environment.
